(19) **Europäisches Patentamt**
**European Patent Office**
**Office européen des brevets**

(11) **EP 1 613 702 B1**

(12) **EUROPÄISCHE PATENTSCHRIFT**

(45) Veröffentlichungstag und Bekanntmachung des Hinweises auf die Patenterteilung:
**14.10.2009 Patentblatt 2009/42**

(21) Anmeldenummer: **04725370.3**

(22) Anmeldetag: **02.04.2004**

(51) Int Cl.:
***C09C 1/64*** (2006.01)

(86) Internationale Anmeldenummer:
**PCT/EP2004/003553**

(87) Internationale Veröffentlichungsnummer:
**WO 2004/087816 (14.10.2004 Gazette 2004/42)**

(54) **DÜNNE DECKENDE ALUMINIUMPIGMENTE, VERFAHREN ZUR HERSTELLUNG DERSELBEN UND VERWENDUNG DER ALUMINIUMPIGMENTE**

THIN COATING ALUMINUM PIGMENTS, METHOD FOR THE PRODUCTION THEREOF, AND USE OF SAID ALUMINUM PIGMENTS

PIGMENTS D'ALUMINIUM À COUVERTURE MINCE, PROCÉDÉ DE FABRICATION ET UTILISATIONS ASSOCIÉS

(84) Benannte Vertragsstaaten:
**AT BE BG CH CY CZ DE DK EE ES FI FR GB GR HU IE IT LI LU MC NL PL PT RO SE SI SK TR**

(30) Priorität: **04.04.2003 DE 10315775**

(43) Veröffentlichungstag der Anmeldung:
**11.01.2006 Patentblatt 2006/02**

(73) Patentinhaber: **Eckart GmbH**
**90763 Fürth (DE)**

(72) Erfinder:
• **SCHLEGL, Thomas**
**91245 Simmelsdorf (DE)**
• **TRUMMER, Stefan**
**90480 Nürnberg (DE)**
• **HENGLEIN, Frank**
**90409 Nürnberg (DE)**
• **SCHNEIDER, Ralph**
**91207 Lauf (DE)**

• **SCHUSTER, Thomas**
**91207 Lauf (DE)**

(74) Vertreter: **Walcher, Armin**
**Louis, Pöhlau, Lohrentz**
**Postfach 3055**
**90014 Nürnberg (DE)**

(56) Entgegenhaltungen:
**EP-A- 0 305 158        EP-A- 0 451 785**
**WO-A-98/17731        US-A- 3 995 815**
**US-A1- 2002 005 144**

• **SEONG-HYEON HONG ET AL.: "Effects of lifter bars on the ball motion and aluminum foil milling in tumbler ball mill" MATERIALS LETTERS, Bd. 57, 2002, Seiten 275-279, XP002305397**
• **S.H. HONG ET AL.: "Fabrication of aluminum flake powder from foil scrap by a wet milling process" MATERIALS LETTERS, Bd. 51, 2001, Seiten 139-143, XP002305398**

**Beschreibung**

Die Erfindung betrifft Aluminiumpigmente, welche wenigstens teilweise mit Schmiermitteln belegt sind sowie ein Verfahren zur Herstellung derselben. Die Erfindung betrifft weiterhin Verwendungen der Aluminiumpigmente.

Aluminiumpigmente sind Effektpigmente und zeichnen sich durch ihr einzigartiges metallisches Aussehen und ihre hohe Deckkraft aus. Aufgrund der plättchenförmigen Struktur dieser Effektpigmente orientieren sie sich im Anwendungsmedium parallel zum Substrat und bewirken durch eine Kombination vieler einzelner Spiegelchen einen metallischen Effekt. Dieser metallische Effekt ist insbesondere in Naßlacken sehr stark ausgeprägt. Dabei handelt es sich bei Volltonlackierungen um einen vom Beobachtungs- und/oder Einfallswinkel abhängigen Helligkeitseffekt, der auch als "Flop" bezeichnet wird. Ein guter Flop wird von vielen Eigenschaften der Pigmente beeinflußt: so spielen ihre Orientierung, ihre Größe und Größenverteilung, ihre Oberflächentextur (Rauheit) und die Kantentextur eine wichtige Rolle.

Die treibende Kraft für eine planparallele Orientierung der Pigmente, die auch als Flakes bezeichnet werden, ist - neben grenzflächenchemischen Unverträglichkeiten der Aluminiumpigmente zum Bindemittelsystem - vor allem der Formfaktor der Pigmente. Unter dem Formfaktor versteht man das Verhältnis von Längsausdehnung d zur Dicke h der Pigmente. Die Längsausdehnung wird vor allem anhand von Laserbeugungsmethoden bestimmt. Dabei wird in der Regel der $d_{50}$-Wert der Summendurchgangskurve herangezogen.

Da die Längsausdehnung der Aluminiumpigmente stark vom jeweiligen Anwendungszweck abhängig ist, kann ein hoher Formfaktor und damit eine möglichst gute Orientierung vor allem über die Dicke der Pigmente erreicht werden. Dünne Pigmente orientieren sich besser und haben daher auch einen höheren Flop.

Eine weitere wichtige Eigenschaft metallischer Coatings oder Druckfarben ist der hohe Glanz. Glanz ist u. a. auch eine physiologisch und psychologisch bedingte Größe, jedoch läßt sich nach der DIN 67 530 das "Glanzvermögen" einer ebenen Oberfläche durch Reflektometerwerte erfassen. Gemessen wird die Reflexion im Glanzwinkel bezogen auf einen Standard (i.d.R. eine schwarze Spiegelglasplatte). Nach dieser Norm werden hochglänzende Proben (Reflektometerwert > 70) unter einem Einfall- bzw. Abstrahlwinkel von 20° und mittelglänzende Oberflächen bei 60° gemessen. Voraussetzung für einen guten Glanz von metallischen Coatings ist ebenfalls eine möglichst gute planparallele Orientierung der plättchenförmigen Pigmente im Anwendungsmedium.

Die brillantesten Aluminiumpigmente mit höchstem Glanz und Flop sind derzeit zwei Klassen zuzuordnen: zum einen so genannte "Silberdollarpigmente", die durch Naßvermahlung von Aluminiumgrieß hergestellt werden, und zum anderen sogenannte "PVD-Pigmente". Silberdollarpigmente zeichnen sich im Vergleich zu Metallpigmenten aus der Zerkleinerungsmahlung durch relativ runde Form und relativ glatte Oberfläche aus.

Durch Naßvermahlung hergestellte Aluminiumpigmente mit hohem Reflektionsgrad und hoher Deckung werden beispielsweise in der EP 0 451 785 B2 beschrieben. Die Pigmente werden charakterisiert durch Wasser-Bedeckungsgrade (Spreitwert) von 2,5 - 5,0 m²/g, einen Rauwert von 2,0 und weniger und einen Formfaktor $d_{50}$/h von 90 und mehr. Aus den Beispielen der EP 0 451 785 B2 sind Formfaktoren bis maximal 140 bekannt.

In der EP 0 451 785 B2 wird auch darauf hingewiesen, daß die Deckkraft von Aluminiumpigmenten nicht nur von ihrer Längsausdehnung, sondern insbesondere auch von ihrer Dicke abhängig ist. Dünnere Pigmente weisen dabei eine höhere Deckkraft auf.

Aluminiumpigmente für Automobillackierungen weisen typischerweise $d_{50}$-Werte von 15 - 20 μm auf. Ein gemäß der Lehre der EP 0 451 785 B2 hergestelltes Aluminiumpigment mit einem Wasser-Bedeckungsgrad von 5,0 m²/g und einem Formfaktor von 90 hätte eine mittlere Dicke h von 80 nm und daher einen $d_{50}$-Wert von 7,2 μm. Ein derartiges Pigment wäre beispielsweise für Automobillackierungen zu klein.

Aluminiumpigmente mit für dieses Marktsegment üblichen $d_{50}$-Werten von 15-20 μm und einem Formfaktor von 90 hätten eine mittlere Dicke h im Bereich von 167 - 222 nm.

EP-A-0305158 EP-A-1424371 und EP-A-1080810 offenbaren Verfahren zur Herstellung von Aluminiumpigmenten durch Vermahlen von Aluminiumpartilkeln unter Verwendung einer Kugelmühle.

In der US 4,318,747 werden feine Aluminiumpigmente mit einer durchschnittlichen Größe von weniger als 5 μm mit leafing-Charakter offenbart, die einen Spreitwert von mindestens 50.000 cm²/g sowie eine spezifische Oberfläche, gemessen nach der BET-Methode, von 24 m²/g bis 93 m²/g besitzen. Aus diesen Angaben können Rauwerte im Bereich von 2,4 bis 9,3 berechnet werden.

Aufgrund der großen Rauheit der Oberfläche dieser Pigmente kommt es zu einer starken Streuung von eingestrahltem Licht und mithin zu einem verringerten Glanz, verglichen mit der glatten Oberfläche eines PVD-Pigments.

Die aus der US 4,318,747 bekannten Aluminiumpigmente sind ferner ebenso wie die aus der EP 0 451 785 B2 bekannten Aluminiumpigmente aufgrund ihrer Feinheit bspw. nicht für die Verwendung in Automobillackierungen geeignet.

Die in der US 4,318,747 angegebenen Beispiele werden stets über lange Mahlzeiten mit Stahlkugeln von 5mm Durchmesser vermahlen. Die Verwendung derartiger Kugeln ist typisch für Zerkleinerungsprozesse.

In der US 3,776,473 werden Aluminiumpigmente mit hoher Reflektivität und glatter Oberfläche sowie runder Form beschrieben. Die in diesem Patent in den Ausführungsbeispielen angeführten Pigmente weisen Wasser-Bedek-

kungsgrade von nur maximal 15.600 cm$^2$/g auf.

Bei PVD-Pigmenten werden extrem dünne (Dicken: 20 bis 50 nm) Al-Pigmente hergestellt. Die Dickenverteilung dieser Pigmente ist äußerst gering. Bei diesem Verfahren wird Aluminium im Ultrahochvakuum auf eine mit einem Ablösefilm ("release-coat") versehene Trägerfolie aufgedampft. Bei diesem Ablösefilm handelt es sich in der Regel um Polymere. Anschließend wird das aufgedampfte Aluminium - soweit möglich - in einem Lösemittel von der Trägerfolie getrennt und die Metallfolie mechanisch oder durch Ultraschall zerkleinert. Die Herstellung von PVD-Pigmenten ist beispielsweise in J. Seubert und A. Fetz, "PVD Aluminium Pigments: Superior Brillance for Coatings and Graphic Arts", Coatings Journal, Bd. 84, A6 225-264, Juli 2001, Seiten 240-245 beschrieben.

Diese PVD-Pigmente weisen aufgrund ihrer extremen Dünnheit ein hervorragendes Deckvermögen auf. Die dünnen Pigmente sind so flexibel, daß sie sich regelrecht an ihrem Untergrund "anschmiegen". Daher sollten sie zur Entfaltung ihrer optischen Möglichkeiten auf einem glatten Untergrund appliziert werden.

Nachteilig bei diesen PVD-Pigmenten sind jedoch die äußerst hohen Produktionskosten des Herstellungsverfahrens. Weiterhin ist von Nachteil, daß der Relase-coat kaum vollständig von den Pigmentteilchen entfernt werden kann. Dieser anhaftende Polymerfilm kann jedoch zu Nachteilen führen. So können in einer Druckfarbe Unverträglichkeiten zum Lösemittel der Druckfarbe auftreten. Beispielsweise können Polymerfilme, die für Toluol geeignet sind, in Lösemitteln wie Alkoholen oder Wasser unverträglich sein. Dies macht sich in der Ausbildung von Agglomeraten bemerkbar, die den gewünschten dekorativen Effekt völlig zunichte machen.

Insbesondere jedoch können derartige polymere Anhaftungen nachteilig stören, wenn man die Aluminiumpigmente nach ihrer Herstellung mit chemischen Schutzüberzügen, wie sie beispielsweise in der DE 196 35 085 beschrieben sind, versieht, um sie korrosionsbeständig zu machen.

Gleiches gilt für eine Stabilisierung durch Korrosionsschutzmittel, wie sie beispielsweise in der DE 100 01 437 beschrieben sind. Hier führen Reste von anhaftendem release-coat zu einer ungleichmäßigen Schutzbeschichtung und verhindern die Aufbringung einer reproduzierbar herstellbaren Schutzschicht. Insbesondere der Einsatz derartig beschichteter Substrate in Wasserlacken, in denen unstabilisierte Aluminiumpigmente eine unerwünschte Gasung durch Vvasserstoffentwicklung hervorrufen, ist mit derart vorbelegten Substraten nicht reproduzierbar erreichbar.

Ein weiterer gravierender Nachteil ist, daß die PVD-Pigmente eine äußerst starke Agglomerationsneigung aufweisen. Aus diesem Grunde werden PVD-Pigmente nur in hochverdünnten Dispersionen mit üblicherweise 10 Gew.-% Aluminiumpigmentanteil angeboten. Im Hinblick auf eine einfachere Handhabung ist es wünschenswert, Präparationen mit höherem Aluminiumpigmentanteil zu haben.

Aufgabe der vorliegenden Erfindung ist es, sehr dünne Aluminiumpigmente ohne anhaftenden Polymerfilm mit ausgezeichneter Deckkraft, hohem Glanz und, verglichen mit aus der herkömmlichen Naßvermahlung erhaltenen herkömmlichen Aluminiumpigmenten, verbessertem metallischen Aussehen bereitzustellen, dem so genannten "Chromeffekt".

Eine weitere Aufgabe der Erfindung ist es, sehr dünne Aluminiumpigmente mit einer gegenüber PVD-Pigmenten deutlich verringerten Agglomerationsneigung bereitzustellen.

Weiterhin sollen derartige Pigmente in einem kostengünstigeren Verfahren, verglichen mit dem aufwendigen PVD-Herstellungsverfahren, hergestellt werden können.

Die Aufgabe wird gelöst durch die Bereitstellung von Aluminiumpigmenten, welche wenigstens teilweise mit Schmiermittel belegt sind,

wobei die Aluminiumpigmente

a) einen Wasser-Spreitwert zwischen 40.000 bis 130.000 cm$^2$/g,

b) eine aus dem Wasser-Spreitwert sowie eine über Dickenauszählung mit Rasterelektronenmikroskopie aus dem $h_{50}$-Wert der Summendurchgangsverteilung errechnete mittlere Dicke h von unter 100 bis 30 nm,

c) eine über Dickenauszählung mit Rasterelektronenmikroskopie ermittelte relative Breite der Dickenverteilung Δh, welche anhand der entsprechenden Summendurchgangskurve der relativen Häufigkeiten nach der Formel

$$\Delta h = 100 \times \frac{h_{90} - h_{10}}{h_{50}}$$ berechnet wird, von 70 % bis 140 %,

d) einen Formfaktor $d_{50}/h$ von über 200,

e) einen Rauwert, der sich aus der spezifischen Oberfläche, welche nach der BET-Methode gemessen wird, und dem Spreitwert nach Maßgabe der folgenden Formel

BET-Wert/(2 x Spreitwert) berechnet wird, von 0,30 bis 0,9 aufweisen.

Bevorzugte Weiterbildungen der erfindungsgemäßen Aluminiumpartikel sind in den Unteransprüchen angebeben.

Die der Erfindung zugrundeliegende Aufgabe wird weiterhin durch ein Verfahren gemäß Anspruch 16 zur Herstellung von Aluminiumpigmenten nach einem der Ansprüche 1 bis 15, gelöst.

Bevorzugte Weiterbildungen des Verfahrens sind in den Unteransprüchen angegeben.

Die erfindungsgemäße Aufgabe wird ferner durch die Verwendung gemäß den Ansprüchen 23 oder 24 sowie einen Nagellack gemäß Anspruch 25 als auch einen Wasserlack gemäß Anspruch 26 gelöst.

Diese Erfindung betrifft Aluminiumpigmente, welche wenigstens teilweise mit Schmiermitteln belegt sind, einen Wasser-Spreitwert von 40.000 bis 130.000 cm$^2$/g, eine aus dem Wasser-Spreitwert sowie über Dickenauszählung mit Rasterelektronenmikroskopie aus dem $h_{50}$-Wert der

Summendurchgangsverteilung errechnete mittlere Dicke h von unter 100 bis 30 nm, eine über Dickenauszählung mit Rasterelektronenmikroskopie ermittelte Breite der Dickenverteilung, die anhand der Summendurchgangskurve nach der Formel $\Delta h = 100 \times \dfrac{h_{90} - h_{10}}{h_{50}}$ berechnet wird, von 70 % bis 140 % sowie einen Formfaktor $d_{50}/h$ über 200 aufweisen.

Bevorzugt sind erfindungsgemäße Aluminiumpigmente mit seinem Wasser-Spreitwert von 45.000 bis 125.000 cm$^2$/g und eine aus dem Wasser-Spreitwert sowie über Dickenauszählung mit Rasterelektronenmikroskopie ($h_{50}$-Wert der Summendurchgangsverteilung) errechnete mittlere Dicke h von unter 89 bis 32 nm. Weiter bevorzugt sind erfindungsgemäße Aluminiu.mpigmente mit einem Wasser-Spreitwert von 50.000 bis 120.000 cm$^2$/g, vorzugsweise 50.000 bis 90.000 cm$^2$/g, und eine aus dem Wasser-Spreitwert sowie über Dickenauszählung mit Rasterelektronenmikroskopie ($h_{50}$-Wert der Summendurchgangsverteilung) errechnete mittlere Dicke h von unter 80 bis 33 nm, vorzugsweise von unter 80 bis 44 nm.

Die erfindungsgemäßen Aluminiumpigmente besitzen aufgrund der geringen Dicke eine sehr hohe Deckkraft.

Einer guten Orientierung von Aluminiumpigmenten steht insbesondere eine schlechte Stapelung der Pigmente in einem Anwendungsmedium entgegen. Um eine möglichst gleichmäßige Stapelung der Pigmente im Anwendungsmedium zu erreichen, sind dünne Pigmente mit einer engen Dickenverteilung sowie eine geringe Pigmentierungshöhe vorteilhaft.

Bei den herkömmlichen dicken Aluminiumpigmenten und bei einer breiten Dickenverteilung kommt es leicht zu Ungleichmäßigkeiten in der Stapelung der Pigmente. So können insbesondere sehr dicke Pigmente als "Abstandshalter" dienen und dann letztlich die Orientierung (Glanz) und die Deckkraft der umgebenden Pigmente beeinträchtigen.

Die erfindungsgemäßen Aluminiumpigmente sind überraschender Weise sehr dünn und weisen zugleich eine enge Dickenverteilung auf. Die erfindungsgemäßen Aluminiumpigmente ähneln in ihren optischen Eigenschaften überraschender Weise den PVD-Pigmenten, werden jedoch - verglichen mit dem aufwendigen PVD-Verfahren - wesentlich einfacher hergestellt und weisen stark verbesserte Handhabungseigenschaften auf, die beispielsweise eine wesentlich höhere Konzentrierung in Präparationen erlauben.

Die exakte mittlere Dicke von plättchenförmigen Metallpigmenten ist nur sehr schwer zu bestimmen. Die DIN 55923 gibt eine Vorschrift zur Messung des Wasserbedeckungsgrades (Spreitung) von "leafing"-Pigmenten an.

Hier wird eine definierte Einwaage von Aluminiumpigmenten in einem leicht flüchtigen organischem Lösemittel auf eine Wasseroberfläche in einer Wanne aufgegeben. Als "leafing"-Pigment ist das Aluminiumpigment beispielsweise mit Stearinsäure belegt und dadurch stark hydrophobiert. Die Pigmente spreiten auf der Wasseroberfläche und bilden einen silbernen Metallfilm. Durch Rühren mit einem Glasstab werden sie zu einem gleichmäßigen "wolkenlosen" Metallfilm verteilt. Anschließend wird der Film durch zwei Lineale zusammengedrückt, bis er erste Falten aufweist. Dann wird der Film wieder soweit entspannt, bis die Falten verschwinden. Die vom Metallfilm bedeckte Fläche wird ausgemessen und anhand der Einwaage des Pigmentes als Spreitwert in cm$^2$/g (oder auch in m$^2$/g) angegeben.

Bei dieser Methode wird unterstellt, das sich die Metallpigmente zumindest im Mittel im Film einzeln nebeneinander anordnen und mithin in einer einzigen "Monolage" Pigment vorliegen.

Anhand dieses Spreitwertes errechnet sich die mittlere Dicke h in nm der Pigmente nach der folgenden Formel:

$$h = \frac{10^7 (nm/cm)}{\rho (g/cm^3) * Spreitwert (cm^2/g)}$$

wobei p die physikalische Reindichte des mit Stearinsäure adsorbierten Pigmentes ist. Hier misst man gewöhnlich einen Wert von ca. 2,5 g/cm$^3$.

In der Norm ist lediglich die Untersuchung von leafing-Pigmenten vorgesehen. Konventionell non-leafing Pigmente können auch nach dieser Methode vermessen werden, wenn sie vor der Spreitung mit Stearinsäure versehen worden sind.

Anhand des Spreitwertes läßt sich lediglich eine mittlere Dicke h der Pigmente bestimmen, über die Breite der Dickenverteilung läßt sich mit dieser Methode jedoch nichts aussagen.

Die Dicke der Pigmente kann ebenfalls mit Hilfe eines

Rasterelektronenmikroskops (REM) bestimmt werden. Hierbei sind so viele Teilchen zu vermessen , daß ein repräsentativer Mittelwert erhalten wird. Üblicherweise werden etwa 100 Teilchen vermessen. Mit diesem Verfahren erhält man - im Unterschied zur Wasserspreitungsmethode - auch einen Überblick über die Dickenverteilung der Pigmente.

Als Mittelwert bietet sich der $h_{50}$-Wert der Dickensummendurchgangskurve an. Ein Maß für die Breite der Verteilung $\Delta h$ wird durch die folgende Formel angegeben:

$$\Delta h(\%) = 100 * \frac{h_{90} - h_{10}}{h_{50}}$$

wobei sich die Indizes auf den jeweiligen Wert der Summendurchgangsverteilung beziehen.

Die erfindungsgemäßen Pigmente besitzen eine relative Breite der Dickenverteilung $\Delta h$ von 70 bis 140%.

Bevorzugt besitzen die erfindungsgemäßen Pigmente eine relative Breite der Dickenverteilung $\Delta h$ von 75 bis 120%.

Unter dem Formfaktor f versteht man das Verhältnis des Mittelwertes der Längsaus-dehnung zur mittleren Dicke der Aluminiumpigmentplättchen.

Die Längsausdehnung d (Durchmesser) wird in Laserbeugungsexperimenten auf Grundlage der Fraunhofer und/oder der Miebeugungstheorie bestimmt. Der Auswertung der Beugungsdaten liegt ein Modell zugrunde, welches auf den Durchmesser einer Äquivalentkugel abzielt. Daher werden keine Absolutwerte erhalten, jedoch haben sich die gemessenen Durchmesser als verlässliche Relativwerte in der Beschreibung der Größencharakteristik von plättchenförmigen Metallpigmente durchgesetzt.

Der dimensionslose Formfaktor f ist dann definiert als:

$$f \doteq 1000 * \frac{d_{50}(\mu m)}{h(nm)}$$

Der $d_{50}$-Wert entspricht dabei 50% der Durchgangssummenverteilungskurve, gemessen und ausgewertet in Form einer Volumenverteilung von Äquivalentkugeln.

Eine weitere Größe zur Pigmentcharakterisierung ist der dimensionslose Rauwert R. Hierunter versteht man das Verhältnis der spezifischen Oberfläche, gemessen nach der BET-Methode (DIN 66132), zu der geometrischen Pigmentoberfläche. Letztere kann unter Vernachlässigung des Randes der Pigmente als der doppelte - Spreitwert berechnet werden:

$$R = \frac{BET - Wert(m^2/g) * 10^4}{2 * Spreitwert(cm^2/g)}$$

Der Rauwert ist ebenfalls als Relativwert zu betrachten, da beide Oberflächenbestimmungsmethoden keine exakten Ergebnisse liefern. Eine ideal glatte Oberfläche sollte theoretisch einen Rauwert von 1 besitzen, tatsächlich findet man mitunter jedoch Werte kleiner als 1.

In der Längsausdehnung unterscheiden sich die erfindungsgemäßen Aluminiumpigmente nicht grundsätzlich von konventionell auf dem Markt befindlichen Aluminiumpigmenten, die durch Naßmahlung hergestellt werden. Im einzelnen hängen die Größen vom Anwendungszweck ab. Die $d_{50}$-Werte der Längenverteilung liegen bevorzugt oberhalb von 6 $\mu$m, weiter bevorzugt in einem Bereich von 6 $\mu$m bis 50 $\mu$m, vorzugsweise von 8 $\mu$m bis 45 $\mu$m, weiter bevorzugt von 12 $\mu$m bis 40 $\mu$m, weiter bevorzugt von 15 $\mu$m bis 30 $\mu$m, noch weiter bevorzugt von 20 $\mu$m bis 25 $\mu$m.

Die erfindungsgemäßen Pigmente zeichnen sich durch einen Formfaktor f über 200 aus. Bevorzugt besitzen die erfindungsgemäßen Pigmente einen Formfaktor f über 220, weiter bevorzugt von mehr ais 240, vorzugsweise von mehr als 300. Gemäß einer weiteren bevorzugten Ausführungsform weisen die Aluminiumpigmente einen Rauwert R von 0,35 bis 0,9, weiter bevorzugt von 0,4 bis 0,8, auf.

Diese Werte zeigen, daß es sich um sehr dünne Pigmente mit relativ glatten Oberflächen handelt.

Charakteristisch für die erfindungsgemäßen Pigmente ist weiterhin ein vergleichsweise niedriger Gehalt an aktivem Aluminium.

Dieser Gehalt läßt sich bestimmen, indem eine definierte Menge Aluminiumpigmente in einer alkalischen Lösung vollständig aufgelöst wird und der entstehende Wasserstoff volumetrisch unter temperierten Bedingungen erfaßt wird. Er liegt bei diesen Pigmenten im Bereich von 85 bis 93, bevorzugt von 87 bis 92%, bezogen auf das Gesamtgewicht der Aluminiumpigmente. Dies ist - im Falle von Aluminiumpigmente aus der Naßmahlung - mit Werten von 93 bis 97% für konventionelle Pigmente zu vergleichen.

Der Restgehalt im Pigment läßt sich dem Aluminiumoxid sowie auf der Oberfläche gebundenen Fettsäuren zuordnen. Aufgrund der geringen Dicke der erfindungsgemäßen Aluminiumpigmente besitzen sie einen vergleichsweise hohen relativen Oxidgehalt. Auch ist der Gehalt an Fettsäuren vergleichsweise hoch. Letzterer läßt sich anhand des C-Gehaltes aus der Elementaranalyse grob abschätzen. Er beträgt bei den erfindungsgemäßen Pigmenten anhand von zuvor mit Aceton oder vergleichbaren Lösemitteln gewaschenen und anschließend getrockneten Aluminiumpulvern typischerweise 0,7 bis 1,5 Gew.%, bevorzugt 0,8 bis 1,4 Gew.-%.

Bei den erfindungsgemäßen Aluminiumpigmenten handelt es sich um sehr dünne Pigmente mit einer engen Dickenverteilung. Derartige Pigmente besitzen eine hohe Deckkraft. Die erfindungsgemäßen Aluminiumpigmente weisen vorzugsweise eine Dickenverteilung mit einem $d_{95}$-Wert von unter 200 nm, vorzugsweise von unter 150 nm auf. Die schmale Dickenverteilung bewirkt vorteilhaft eine sehr gute Stapelung der Pigmente in einem Anwendungsmedium, beispielsweise einem Lack oder einer Druckfarbe. Mit den erfindungsgemäßen Pigmenten können beispielsweise deckende Lackierungen mit sehr geringen Schichtdicken, beispielsweise einer Schichtdicke von weniger als 10 $\mu$m, mit sehr hohem Glanz und sehr gutem Flop hergestellt werden.

Insbesondere in der Automobillackierung besteht ein Bedürfnis nach geringeren Schichtdicken. Die treibende Kraft ist hier vor allem eine Kosteneinsparung. Bislang liegen Basislackschichtdicken in einem typischen Bereich von 15 $\mu$m. Bereits jetzt werden an sehr gebogenen Formkörper wie beispielsweise Türklinken standardmäßig auch geringere Schichtdicken verwendet. Es wäre wünschenswert, wenn niedrige Schichtdicken bis hin zu unter 10 $\mu$m verwirklicht werden könnten. Allerdings darf die Schichtdicke nicht zu niedrig sein, da andernfalls Haftungs-, Deckungs- und/oder Pigmentierungsprobleme auftauchen.

Die erfindungsgemäßen Pigmente eignen sich äußerst vorteilhaft für die Verwendung in Beschichtungsmittel, die in sehr dünnen Schichtdicken aufgebracht werden sollen.

Im folgenden wird auf das Verfahren zur Herstellung der erfindungsgemäßen Aluminiumpigmente eingegangen. Dieses zeichnet sich durch eine äußerst schonende Verformungsmahlung von Aluminiumpartikeln aus. Im einzelnen besteht das Verfahren aus folgenden Schritten:

Die Aluminiumpartikel werden unter Verwendung eines Mahlwerks, wobei das Mahlwerk eine Topfmühle, eine Kugelmühle, Rührwerkskugelmühle oder eines Attritors ist, in Gegenwart von Lösemittel und Schmierstoffen als Mahlhilfsmittel und von Mahlkörpern, die ein Einzelgewicht von 2 bis 13 mg aufweisen, über einen Zeitraum, von etwa 15 bis etwa 72 Stunden, vermahlen.

Gemäß einer bevorzugten Weiterbildung der Erfindung weisen die Mahlkörper ein Einzelgewicht von 5,0 bis 12,0 mg auf. Als Mahlkörper werden vorzugsweise sphärische Körper, weiter bevorzugt Kugeln verwendet.

Nach der Vermahlung der Aluminiumpartikel werden die erhaltenen Aluminiumpigmente von den Mahlkörpern, vorzugsweise den Mahlkugeln, abgetrennt. In einem weiteren Verfahrensschritt können die erhaltenen Aluminiumpigmente einer Größenklassifikation unterzogen werden. Nachfolgend können die Aluminiumpigmente in eine geeignete Angebotsform überführt werden. Beispielsweise können die klassierten oder nicht-klassierten Aluminiumpigmente in eine Pulverform, vorzugsweise in eine nichtstaubende Pulverform, überführt werden. Die Aluminiumpigmente können aber auch durch Kompaktierung in eine Paste, Granulate, Pellets, überführt werden.

Unter Pellets werden im Sinne der Erfindung auch Briketts, Tabletten oder Würstchen verstanden.

Die Pelletierung kann auf einem Pelletierteller auf herkömmliche Art und Weise durchgeführt werden. Das Tablettieren kann in einer Tablettiervorrichtung erfolgen. Die Würstchen können durch ein Preßverfahren aus Aluminiumpaste oder -pulver hergestellt werden oder indem eine Aluminiumpaste durch einen Extruder extrudiert wird und die extrudierten Pastenstränge durch eine umlaufende Messeranordnung zerteilt werden. Ein Granulieren der erfindungsgemäßen Aluminiumpigmente kann beispielsweise durch Sprühgranulieren erfolgen.

Die erfindungsgemäßen Aluminiumpigmente können äußerst vorteilhaft in Granulaten oder Pellets mit hohen Aluminiumpigmentgehalten, beispielsweise von 98 Gew.-% bis 50 Gew.-%, vorzugsweise von 95 Gew.-% bis 70 Gew.-%, bereitgestellt werden. Die vorgenannten Präparationen lassen sich beispielsweise in Lacksysteme oder Druckfarben sehr gut einarbeiten, ohne daß es zu unerwünschten Agglomerationen von Aluminiumpigmenten kommt

Die Mahlung kann in einem Lösemittel bei einem Gewichtsverhältnis von Lösemittel zu Aluminiumpartikel von 2,8 bis 10 und bei einem Gewichtsverhältnis der Mahlkugeln zu Aluminiumpartikeln von 20 - 70 und mit Schmierstoffen als Mahlhilfsmittel stattfinden.

Die kritische Drehzahl n$_{krit}$ ist ein wichtigen Parameter, der angibt, ab wann die Kugeln durch die Zentrifugalkräfte an die Mühlenwand gepresst werden und praktisch keine Mahlung mehr stattfindet:

$$n_{krit} = \sqrt{\frac{g}{2\pi^2} \bullet \frac{1}{D}}$$

wobei D der Trommeldurchmesser
und g die Gravitationskonstante ist.

Die Umdrehungsgeschwindigkeiten der Kugelmühle betragen 50 % bis 62% der kritischen Drehzahl n$_{krit}$.

Niedrige Umdrehungsgeschwindigkeiten begünstigen eine langsame Verformung der Aluminiumpartikel. Um eine langsame Verformung zu bewirken, werden bei dem erfindungsgemäßen Verfahren bevorzugt auch leichte Mahlkugeln verwendet. Mahlkugeln mit einem Einzelgewicht über 13 mg verformen die Aluminiumpartikel zu stark, was zu vorzeitigem Bruch führt. Als Aluminiumpartikel wird vorzugsweise Aluminiumgrieß verwendet.

Die oben angeführten Bedingungen führen zu einer sehr schonenden Mahlung, bei der die Aluminiumpartikel langsam ausgeformt werden und Brüche infolge eines Kugelstoßes mit hoher kinetischer Energie vermieden werden. Aufgrund der äußerst schonenden Mahlweise dauert diese Art der Mahlung vergleichsweise lang. Die Mahlzeit beträgt 15 bis 72 h, bevorzugt 16 bis 50 h.

Die langen Mahlzeiten führen zu einer Vielzahl von Pigment-Kugel-Stößen. Dadurch wird das Pigment sehr gleichmäßig ausgeformt, was sich in einer sehr glatten Oberfläche und in einer engen Dickenverteilung bemerkbar macht.

Im Unterschied zu herkömmlichen Mahlverfahren werden die Aluminiumpartikel bei dem erfindungsgemäßen Verfahren zum überwiegenden Anteil nicht vermahlen bzw. zerkleinert, sondern äußerst schonend über einen längeren Zeitraum verformt.

Bei dem verwendeten Mahlgut aus Aluminium handelt es sich bevorzugt um Aluminiumgrieß. Dieser Aluminiumgrieß wird bevorzugt in "Atomizern" durch Verdüsung von flüssigem Aluminium hergestellt. Auch Folienpulver aus einer Aluminiumfolie sowie Abfallfolien können Verwendung finden. Der Grieß kann eine runde oder spratzige Form haben. Aluminiumpartikel in Nadelform werden bei dem erfindungsgemäßen Verfahren als Ausgangsmaterial nicht verwendet, da diese nicht zu dünnen Effektpigmenten vermahlbar sind. Bevorzugt ist, daß die Aluminiumpartikel eine kugelförmige bis ellipsoide Form aufweisen.

Der Aluminiumgrieß sollte vorzugsweise einen mittleren Durchmesser von unter 10 $\mu$m haben. Die Reinheit des verwendeten Aluminiums beträgt vorzugsweise 99,0 bis über 99,5%.

Als Schmierstoffe können eine Vielzahl von Verbindungen verwendet werden. Hierbei sind die schon seit langer Zeit verwendeten Fettsäuren mit Alkylresten von 10 bis 24 C-Atomen zu nennen. Vorzugsweise werden Stearinsäure, Ölsäure oder Mischungen derselben verwendet. Dabei führt Stearinsäure als Schmiermittel zu leafing-Pigmenten, Ölsäure hingegen zu non-leafing-Pigmenten. Leafing-Pigmente sind dadurch gekennzeichnet, daß sie in einem Anwendungsmedium, bspw. einem Lack oder eine Druckfarbe aufschwimmen, d.h. sich an der Oberfläche des Anwendungsmediums anordnen. Non-leafing-Pigmente ordnen sich hingegen im Anwendungsmedium an. Den Fettsäuren können zusätzlich beispielsweise langkettige Aminoverbindungen zugesetzt werden. Die Fettsäuren können tierischen oder auch pflanzlichen Ursprungs sein. Ebenfalls können organische Phosphonsäuren und/oder Phosphorsäureester als Schmiermittel verwendet werden.

Das Schmiermittel sollte nicht in zu geringer Menge eingesetzt werden, da anderen-falls infolge der starken Ausformung der Aluminiumpartikel die sehr großen Oberflächen der hergestellten plättchenartigen Aluminiumpigmente nur ungenügend durch adsorbiertes Schmiermittel abgesättigt werden. In diesem Fall kommt es zu Kaltverschweißungen. Typische Mengen sind daher 1 bis 20 Gew.-%, bevorzugt 2 bis 15 Gew.-%, Schmiermittel bezogen auf das eingesetzte Aluminiumgewicht.

Die Wahl des Lösemittels ist an sich unkritisch. Man kann übliche Lösemittel wie Testbenzin, Solvent Naphtha etc. einsetzen. Auch die Verwendung von Alkoholen, wie z.B. Isopropanol, Ether, Ketone, Ester usw. ist möglich.

Ebenfalls kann Wasser (im zumindest überwiegenden Teil) als Lösemittel verwendet werden. In diesem Fall sollten die eingesetzten Schmiermittel allerdings deutlich korrosionsinhibierende Wirkung haben. Bevorzugt sind hier. Phosphonsäuren und/oder Phosphorsäureester, die auch ethoxylierte Seitenketten tragen können. Auch die Zugabe von Korrosionsinhibitoren während der Mahlung ist hier vorteilhaft.

Die verwendeten Kugeln weisen ein Einzelgewicht von 2 bis 13 mg aufweisen. Weiterhin ist bevorzugt, daß die verwendeten Kugeln ein Einzelgewicht von 5,0 bis 12,0 mg aufweisen. Bevorzugt sind Kugeln mit glatter Oberfläche, möglichst runder Form und einheitlicher Größe. Das Kugelmaterial kann aus Stahl, Glas oder Keramik, wie z.B. Zirkoniumoxid oder Korund sein.

Die Temperaturen während des Mahlvorganges liegen im Bereich von 10°C bis 70 °C. Bevorzugt sind Temperaturen in einem Bereich von 25 °C bis 45 °C.

Bedingt durch das erfindungsgemäße Herstellungsverfahren sind die erfindungsgemäßen Aluminiumpigmente äußerst vorteilhaft frei von anhaftenden Polymerfolien. Daher besitzen die erfindungsgemäßen Aluminiumpimente nicht die Nachteile von noch mit Resten des "Release-coats" behafteter Aluminiumpigmente, die durch PVD-Verfahren hergestellt werden. Zudem ist ihre Herstellungsweise billiger als die aufwendigen PVD-Herstellungsverfahren. Die Trennung der hergestellten Aluminiumpigmente von den Mahlkörpern, vorzugsweise Mahlkugeln, kann auf herkömmliche Art und Weise durch Siebung erfolgen.

Nach der Abtrennung von den Mahlkugeln werden die Aluminiumpigmente vorzugsweise einer Größenklassifikation unterzogen. Diese Klassifikation sollte schonend durchgeführt werden, um die dünnen Aluminiumpigmente nicht zu zerstören. Es kann sich dabei beispielsweise um eine Naßsiebung, eine Dekantierung oder auch einer Trennung durch Sedimentation handeln. Bei der Naßsiebung wird i.d.R. der Grobanteil herausgesiebt. Bei den anderen Verfahren kann insbesondere der Feinstanteil abgetrennt werden. Anschließend wird die Suspension von überschüssigem Lösemittel getrennt (z.B. mit Hilfe einer Filterpresse).

Im letzten Schritt erfolgt eine Weiterverarbeitung zur gewünschten Angebotsform. Dies kann das Ergänzen mit Lösemittel zu einer Paste beinhalten oder das Austrocknen zu Pulver.

Das getrocknete Pulver kann durch Zugabe sehr kleiner Mengen Lösemittel (<10%) in einem geeigneten Homogenisator zu einem nichtstaubenden Metallpulver weiterverarbeitet werden. Auch kann der Filterkuchen zunächst ausgetrocknet und anschließend mit einem anderen Lösemittel wieder angepastet werden (Umnetzen).

Schließlich können die erfindungsgemäßen Pigmente durch Versetzen des Filterkuchens mit einer geeigneten Dispersion eines geeigneten Harzes zu Pellets, Granulaten oder Tabletten weiterverarbeitet werden. Diese Angebotsformen besitzen die Vorteile, daß sie nicht stauben, eine leichte Dosierbarkeit aufweisen und hervorragend dispergierbar sind.

Aufgrund der recht hohen spezifischen Oberfläche der erfindungsgemäßen Aluminiumpigmente müssen beispielsweise zur Pelletierung der erfindungsgemäßen Aluminiumpigmente relativ große Mengen an Dispergierharz verwendet werden.

Vorzugsweise werden 2-50 Gew.-%, weiter bevorzugt 5 bis 30 Gew.-%, Harz, bezogen auf die Gesamtformulierung des Pellets verwendet.

Zur Pelletierung kann eine Vielzahl von Dispergicrharzen verwendet werden. Beispiele hierfür sind sowohl natürlich vorkommende wie auch synthetische Harze. Sie umfassen beispielsweise Alkydharze, Carboxymethyl- und Carboxyethylcelluloseharze, Cellulose Acetat, Cellulose Acetat Propionat (CAP) und Cellulose Acetat Butyrat (CAB), Cumarol-Indenharze, Epoxidester, Epoxid-Melamin und Epoxid-Phenol-Kondensate, Ethyl- und Methylcellulose, Ethylhydroxyethyl-cellulose, Hydroxyethylcellulose, Hydroxypropylmethylcellulose, Ketone und Maleinsäureharze, Melaminharze, Nitrocelluloseharze, Phenol- und modifizierte Phenolharze, Polyacrylamid-, Polycarbonat-, Polyamid-, Polyester-, Polyether-, Polyurethan-, und Vinylharze.

Unter diesen polymeren Harzen sind insbesondere zu erwähnen: acrylatische Copolymere und Acrylesterharze, Polyacrylonitril- und Acrylonitrilcopolymerharze, Copolymere aus Butadien und Vinylidenchloride, Butadien/Styrol Copolymere, Methylacrylat- und Methymethacrylatcopolymere; sowie Polybuten-, Polyisobutylen-, Polyvinylacetat-, Polyvinylakohol-, Polyvinylchlorid-, Polyvinylether-, Polyvinylpyrrolidon- und Polystyrolharze. Weitere Copolymere beinhalten Styrol/Maleinsäureanhydrid- und Styrol/Schellackharze, Vinylchlorid/Vinylacetat-, Vinylchlorid/Vinylether- und Vinylchlorid/Vinylidenchloridharze.

Ferner kommen natürlich vorkommende Harze wie Gummi Arabicum, Gutta Percha, Casein und Gelatine in Betracht.

Bevorzugt sind Aldehydharze wie die Laropalserie der BASF AG, Ludwigshafen. Weiterhin kommen Wachse als Bindermaterialien in Frage. Hier sind als Beispiele natürliche Wachse wie Bienenwachs, Candelilla-, Camauba-, Montan- sowie -

Parafinwachse zu nennen. Ebenso kommen synthetische Wachse wie beispielsweise PE-Wachse in Betracht.

Es hat sich überraschend herausgestellt, daß die Agglomerationsneigung der erfindungsgemäßen Aluminiumpigmente deutlich geringer ist als die von PVD-Pigmenten.

Es wird vermutet, daß dieser Effekt neben der Dicke der Pigmente auch mit der Dickenverteilung und der Rauheit der erfindungsgemäßen Aluminiumpigmente verbunden ist. Bei einer Dickenverteilung im Bereich von 70 % bis 140 % tritt eine stark verringerte Agglomerationsneigung auf. Des weiteren weisen die erfindungsgemäßen Aluminiumpigmente aufgrund des Herstellungsverfahrens ein gewisses Maß an Rauheit bzw. Welligkeit auf, die eine planparallele Anlagerung, d.h. eine Agglomeration von Aluminiumpigmenten aneinander verhindert, ohne daß es überraschenderweise zu einer wesentlichen Beeinträchtigung der , optischen Eigenschaften, wie Reflexionsvermögen und Glanz, der erfindungsgemäßen Aluminiumpigmente kommt.

Im Unterschied zu PVD-Pigmenten weisen übereinandergelagerte erfindungsgemäße Aluminiumpigmente aufgrund der Rauheit bzw. Welligkeit nur punktförmige Kontaktflächen zueinander auf. Dadurch wird - im Unterschied zu PVD-Pigmenten - die Ausbildung kurzreichender Anziehungskräfte wie van der Waals-Kräfte oder Wasserstoffbrücken minimiert und mithin eine Agglomeration oder Aggregation erschwert.

In einer weiteren erfindungsgemäßen Ausführungsform werden die erfindungsgemäßen Aluminiumpigmente nachträglich mit einer passivierenden Inhibitor-und/oder Korrosionsschutzschicht belegt bzw. beschichtet. Derartige Beschichtungen ermöglichen erst den sicheren Einsatz der erfindungsgemäßen Pigmente in Wasserlacken und/oder in Außenanwendungen.

Der Wirkungsmechanismus der Passivierungsschichten ist komplex. Bei Inhibitoren beruht er zumeist auf sterischen Effekten. Der größte Teil der Inhibitoren hat daher auch eine orientierende Wirkung im Sinne von "leafing" und "non-leafing", d.h. im Medium aufschwimmend bzw. nicht aufschwimmend.

Die Inhibitoren werden i.a. in niedrigen Konzentrationen in der Größenordnung von 0,5 Gew.-% bis 15 Gew.-% bezogen auf das Gewicht des eingesetzten Aluminiumpigmentes zugegeben.

Für die Inhibierung kommen vorzugsweise in Frage:

Organisch modifizierte Phosphonsäuren der allgemeinen Formel R-P(O)(OR$_1$)(OR$_2$), wobei: R = Alkyl Aryl, Alkyl-aryl, Aryl-alkyl sowie von Alkylether, insbesondere ethoxylierte Alkylether und R$_1$, R$_2$ = H, C$_n$H$_{2n+1}$, mit n = 1-6 ist, wobei Alkyl jeweils verzweigt oder unverzweigt sein kann. R$_1$ kann gleich oder unterschiedlich zu R$_2$ sein.

Organisch modifizierte Phosphorsäuren und -ester der allgemeinen Formel R-O-P(OR$_1$)(OR$_2$) mit R = Alkyl , Aryl, Alkyl-aryl, Aryl-alkyl sowie von Alkylether, insbesondere ethoxylierte Alkylether und R$_1$, R$_2$, = H, C$_n$H$_{2n+1}$, mit n = 1-6 ist, wobei Alkyl jeweils verzweigt oder unverzweigt sein kann.

Verwendet werden können reine Phosphonsäuren oder -ester oder Phosphorsäuren oder -ester oder beliebige Mischungen derselben.

Im Falle einer Vermahlung der Aluminiumpartikel in überwiegend wäßrigem Lösemittel werden derartige Inhibitoren als Mahlhilfsmittel verwendet, um einer sicherheitstechnisch gefährlichen Wasserstoffentstehung während des Mahlvorganges vorzubeugen.

Weiterhin kann die passivierende Inhibitorschicht korrosionsinhibierenden organisch funktionalisierten Silanen, aliphatischen oder cyclischen Aminen, aliphatischen oder aromatischen Nitroverbindungen, Sauerstoff-, Schwefel und/ oder Stickstoff enthaltende Heterocyclen wie beispielsweise Thioharnstoffderivaten, Schwefel und/oderStickstoffverbindungen höherer Ketone, Aldehyden und Alkoholen (Fett-alkoholen), Thiolen, β-Ketoestem, β-Diketonen oder Gemischen derselben bestehen oder umfassen. Die passivierende Inhibitorschicht kann aber auch aus den vorgenannten Substanzen bestehen. Bevorzugt sind organische Phosphonsäuren und/oder Phosphorsäureester oder deren Gemische.

Die Passivierung über Korrosionsschutzbarneren mit chemischer und physikalischer Schutzwirkung ist auf vielfältige Weise realisierbar.

Passivierende Korrosionsschutzschichten, die den Aluminiumpigmenten einen besonders guten Korrosionsschutz gewährleisten, umfassen oder bestehen aus Siliciumoxid, Chromoxid, das vorzugsweise durch Chromatierverfahren aufgebracht wird, Zirkoniumoxid, Aluminiumoxid, polymerisierte Kunststoffharze, Phosphat, Phosphit oder Borat oder Mischungen derselben.

Bevorzugt sind Siliciumoxidschichten und Chromoxidschichten (Chromatierung). Die SiO$_2$-Schichten werden bevorzugt durch Sol-Gel-Verfahren mit Schichtdicken von 20 - 150 nm in organischen Lösemitteln hergestellt.

Die erfindungsgemäßen Aluminiumpigmente finden Verwendung in Coatings, Lacken, Druckfarben, Pulverlacken, Kunststoffen und kosmetischen Formulierungen. Vorzugsweise werden die erfindungsgemäßen Aluminiumpigmente in Nagellackformulierungen verwendet. Der erfindungsgemäße Nagellack besitzt ein äußerst metallisches Aussehen.

Die durch nachträgliche Beschichtungen passivierten erfindungsgemäßen Aluminiumpigmente finden bevorzugt Verwendung in Wasserlacken und Außenanwendungen. Der erfindungsgemäße Wasserlack enthält neben den passivierten erfindungsgemäßen Aluminiumpigmenten die üblichen mit Wasser kompatiblen Bindemittel wie Polyester, Polyacrylate, Polymethacrylate und/oder Polyurethane, etc..

Die folgenden Beispiele erläutern die Erfindung, ohne sie einzuschränken.

**Erfindungsgemäße Beispiele 1 bis 3:**

Beispiel 1: In einer Topfmühle (Länge: 32 cm, Breite: 19 cm) werden 3,1 kg Glaskugeln (Durchmesser: 2 mm), 310 g Testbenzin, 93 g Aluminiumgrieß (mittlerer Durchmesser < 8 μm) und 9,3 g Ölsäure aufgegeben. Anschließend wird 20 h lang bei 57 U/min vermahlen. Das Produkt wird durch Spülen mit Testbenzin von den Mahlkugeln getrennt und anschließend in einer Naßsiebung auf einem 25 μm-Sieb durchgesiebt. Das Feinkorn wird über einer Nutsche weitgehend von Testbenzin befreit und anschließend mit Testbenzin in einem Labormischer angepastet (ca. 70% Feststoffanteil).

Beispiel 2: Vermahlung wie in Beispiel 1, jedoch mit einem Grieß mit einem mittleren Durchmesser < 6 μm und einer Mahldauer von 23 h.

Beispiel 3: In einer Topfmühle (Länge: 32 cm, Breite: 19 cm) werden 5,0 kg Stahlkugeln (Durchmesser: 1,1 mm, Gewicht 5,5 mg), 160 g Testbenzin, 150 g Aluminiumgrieß (mittlerer Durchmesser < 8 $\mu$m) und 6 g Ölsäure aufgegeben. Anschließend wird 16 h lang bei 60 U/min vermahlen. Das Produkt wird durch Spülen ausreichend Testbenzin von den Mahlkugeln beim Sieben getrennt und anschließend in einer Naßsiebung auf einem 25 $\mu$m-Sieb durchgesiebt. Das Feinkorn wird über einer Nutsche weitgehend von Testbenzin befreit und anschließend mit Testbenzin in einem Labormischer angepastet (ca. 70% Feststoffanteil).

Vergl. Beispiel 4: Metalure L 55300 (Fa. Eckart)
Vergl. Beispiel 5: MH 8801 (Fa. Asahi)
Vergl. Beispiel 6: MH 9901 (Fa. Asahi)
Vergl. Beispiel 7: VP 53534 (Fa. Eckart), Silberdollarpigment
Vergl. Beispiel 8: MEX 2192 (Fa. Eckart), Silberdollarpigment

Die Proben der erfindungsgemäßen Beispiele sowie der ausgewählten Vergleichsbeispiele wurden durch Spreitwerte charakterisiert und hieraus mittlere Dicken berechnet.

Die Spreitwertbestimmung wurde in Anlehnung an die DIN 55923 durchgeführt. Dabei wurden in Ergänzung zu dieser Norm, die nur für leafing-Pigmente gilt, non-leafing Pigmente vor der Spreitung durch folgende Behandlung in leafing-Pigmente überführt: 200 mg Aluminiumpigmente werden in Form einer Paste oder eines Filterkuchens abgewogen und in einer Lösung aus 3,15 g Stearinsäure und 63 ml Testbenzin dispergiert und für 12 min im Ultraschallbad behandelt. Anschließend wird die Dispersion auf einer Glasfritte abgesaugt, 3x mit Aceton nachgewaschen, trockengesaugt und im Vakuumexsikkator getrocknet. Im Normalfall werden dann ca. 4 mg des mit Stearinsäure aufgefetteten Aluminiumpulvers mit wenigen Tropfen n-Butanol auf einem Uhrdeckelgläßchen mit einem Glasstab angepastet und dann die gesamte Paste in die Spreitwanne aufgegeben.

Bei den sehr dünnen erfindungsgemäßen Aluminiumpigmenten ergab sich jedoch eine Besonderheit bei der Probenpräparation. Hier waren die Spreitwerte so hoch, daß der Metallfilm zu groß für die der DIN 55923 entsprechenden Wasserwanne war. Entsprechend dimensionierte Metallspiegel konnten nur durch Einwaage von 1 mg Probenmenge erreicht werden. In diesem Fall jedoch macht sich in den Meßwerten eine deutliche Varianz (> 10%) bemerkbar, die durch den entstehenden Einwaagefehler bedingt ist.

Alternativ wurde bei diesen Proben mit mit Stearinsäure aufgefettetem Aluminiumpulver eine Dispersion in n-Butanol hergestellt. Die Konzentration war so dimensioniert, daß 1 ml dieser Dispersion ca. 1 mg Pigment enthielt. Zur Probenentnahme wurde unter starkem Rühren zur Vermeidung von Absetzbewegungen der Pigmentteilchen ca. 1 ml Dispersion mittels einer skalierten Pipette entnommen und die Dispersion vorsichtig auf die Spreitwanne verteilt. Die relative Varianz der Spreitwerte lag bei dieser Methode unter 10%.

Die Proben der erfindungsgemäßen Beispiele 1-3 sowie ausgewählte Vergleichsbeispiele wurden zur näheren Bestimmung der Teilchendicken unter Verwendung eines Feldionen-Rasterelektronenmikroskops charakterisiert.

Für die REM-Untersuchung wurde die Proben, wie nachstehend beschrieben, präpariert:

a) Erfindungsgemäße Aluminiumpigmente und herkömmliche Pigmente aus Naßmahlung

Die erfindungsgemäßen Aluminiumpigmente bzw. die herkömmlichen, aus einer üblichen Naßvermahlung erhaltenen Aluminiumpigmente, die jeweils als Paste oder Filterkuchen vorliegen, werden jeweils zunächst mit Aceton gewaschen und dann ausgetrocknet.

Ein in der Elektronenmikroskopie übliches Harz, beispielsweise TEMPFIX (Gerhard Neubauer Chemikalien, D-48031 Münster, Deutschland), wird auf einen Probenteller aufgebracht und auf einer Heizplatte bis zum Erweichen erhitzt. Nachfolgend wird der Probenteller von der Heizplatte genommen und das jeweilige Aluminiumpulver auf das erweichte Harz gestreut. Das Harz wird durch die Abkühlung wieder fest und die aufgestreuten Aluminiumpigmente können-bedingt durch das Wechselspiel zwischen Adhäsion und Schwerkraft- nahezu senkrecht stehend und fixiert auf dem Probenteller präpariert werden. Dadurch sind die Pigmente im Elektronenmikroskop seitlich gut zu vermessen. Bei der Vermessung der Dicke wird der azimuthale Winkel $\alpha$ des Pigmentes zu einer zur Oberfläche normalen Ebene geschätzt und bei der Dickenauswertung nach der Formel

$$h_{eff} = h_{mess}/\cos\alpha$$

berücksichtigt. Von den $h_{eff}$-Werten wurde anhand der relativen Häufigkeiten die Summenverteilungskurve erstellt. Es werden mindestens ca. 100 Teilchen gezählt.

b) PVD-Pigmente

Eine PVD-Pigmentsuspension wurde mit einem großen Überschuß Aceton mehrmals gewaschen, um sie weitgehend von Resten des release-coats zu befreien. Anschließend wurden die-PVD-Pigmente in Aceton dispergiert und ein Tropfen der Dispersion auf ein Mikroskopigläschen verteilt. Nach dem Verdunsten des Lösemittels wird das Glas zerschnitten. Die einzelnen Scherben können senkrecht stehend in das Elektronenmikroskop eingespannt werden. Bei scharfen Bruchkanten können genügend PVD-Pigmente vermessen werden. Hier reichen aufgrund der schmalen Dickenverteilung ca. 50 Teilchen zur Erzielung aussagekräftiger Ergebnisse.

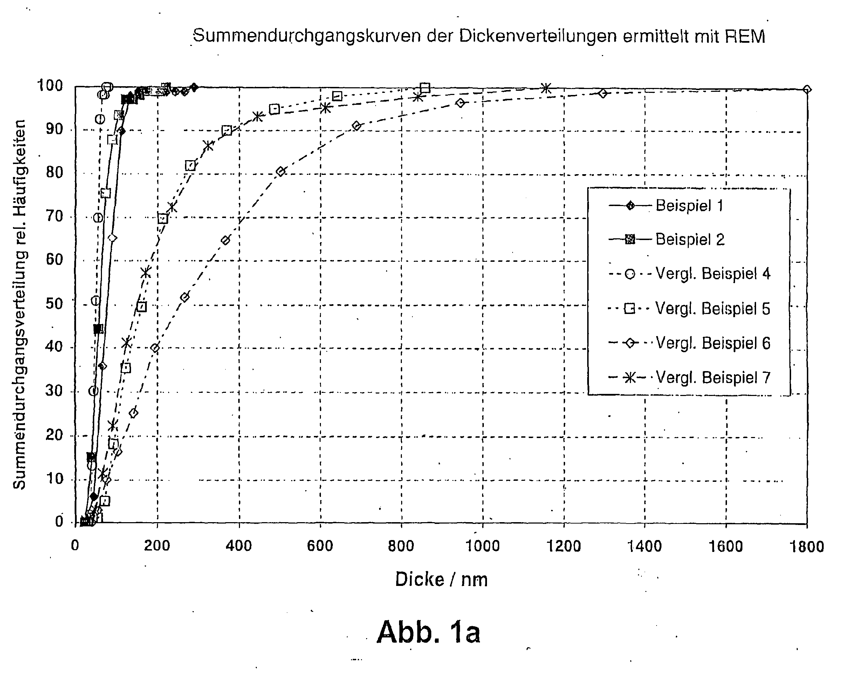

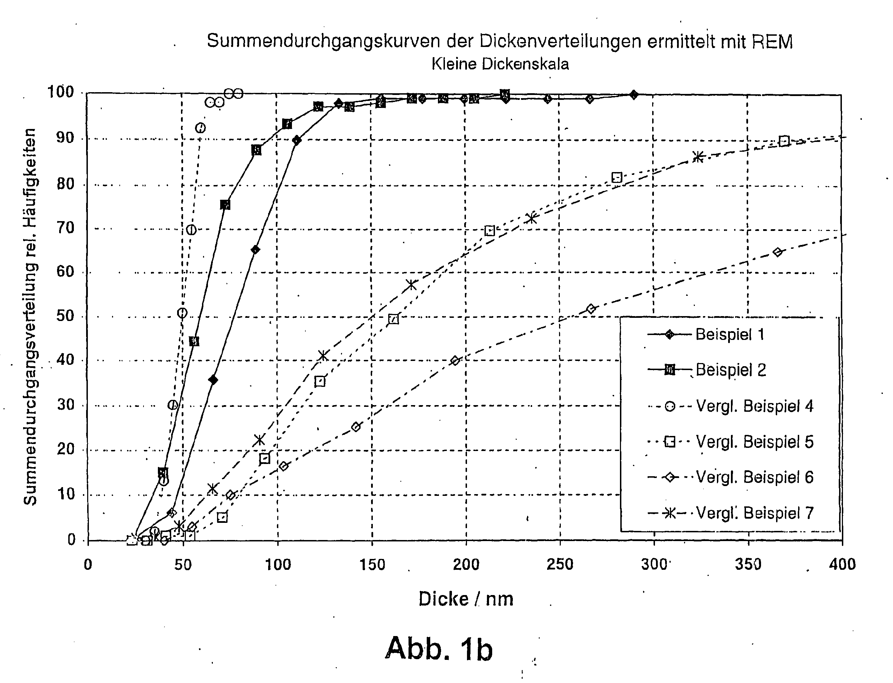

Die Summendurchgangsverteilungen der Dickenverteilung der verschiedenen erfindungsgemäßen Proben und Vergleichs-Proben sind in Abb.1 a und b dargestellt. Die Kurven in Abb. 1 b stellen einen vergrößerten Ausschnitt der Kurven in Abb. 1a dar. Die Zahl der vermessenen Teilchen lag bei 50 (PVD-Pigmente) bis 192 (konventionelle Pigmente). Statistische Analysen ergaben, daß die Summendurchgangskurve ab 75 bis 100 Teilchen für die erfindungsgemäßen Pigmente und konventionelle Pigmente aus der Naßmahlung weitgehend konstant war.

Die Ergebnisse sind in Tab. 1 zusammengefasst.

**Tabelle 1: Physikalische Charakterisierung**

| Probe | Cilas: $d_{50}$ [μm] | Spreitwert [cm²/g] | Mittl. Dicke $h_{Spreitung}$ [nm] | REM-Statistik | | BET [m²/g] | Aktiver Al -Gehalt | Rauwert (Spreitung) | Formfaktor (Spreitung) |
|---|---|---|---|---|---|---|---|---|---|
| | | | | $h_{50}$ [nm] | $h_{90}$-$h_{10}$/$h_{50}$ | | | | |
| Beispiel 1 | 19 | 50.100 | 80 | 78 | 84% | 6,6 | 92,2% | 0,69 | 238 |
| Beispiel 2 | 19 | 84.600 | 47 | 60 | 97% | 7,6 | 91,1% | 0,45 | 402 |
| Beispiel 3 | 22 | 52.900 | 76 | 79 | 104% | 7,2 | 92,8% | 0,68 | 291 |
| Vergl. Beispiel 4 | 12,5 | -- | -- | 49 | 41% | -- | -- | -- | 255 |
| Vergl. Beispiel 5 | 17 | 30.000 | 133 | 163 | 180 % | | -- | | 128 |
| Vergl. Beispiel 6 | 22 | 24.800 | 161 | 259 | 221% | | -- | | 137 |
| Vergl. Beispiel 7 | 17,5 | 31.700 | 126 | 148 | 210% | 4,5 | 96,2% | 0,71 | 139 |
| Vergl. Beispiel 8 | 14,5 | 20.700 | 193 | -- | -- | 5,6 | 94,8% | 1,35 | 72 |
| Beispiel 1: Erfindungsgemäßes Beispiel<br>Beispiel 2: Erfindungsgemäßes Beispiel<br>Beispiel 3: Erfindungsgemäßes Beispiel<br>Vergl. Beispiel 4: Metalure L55350 (Eckart) PVD-Pigment<br>Vergl. Beispiel 5: MH 8801 (Asahl)<br>Vergl. Beispiel 6: MH 9901 (Asahl)<br>Vergl. Beispiel 7: VP 53534 (Eckart), Silberdollarpigment<br>Vergl. Beispiel 8: MEX 2192 (Fa. Eckart), Silberdollarpigment | | | | | | | | | |

Generell ergibt sich eine gute Übereinstimmung mit der aus der Spreitwertmethode ermittelten mittleren Dicke $h_{Spreitung}$ und dem Medianwert der

Summendurchgangskurve der Dickenverteilung $h_{50}$. Ebenfalls aufgeführt sind die BET-Oberflächen, $d_{50}$-Werte der Größenverteilung sowie die berechneten Rauwerte R und Formfaktoren f der Pigmente. Für die Berechnung dieser Werte wurden - wie üblich - die Spreitwerte bzw. daraus berechnete durchschnittliche Dicken herangezogen.

Die Längsausdehnung d wurde mit Hilfe eines Lasergranulometers (Cilas 1064, Firma Cilas, Frankreich) bestimmt und als Maß der mittleren Längsausdehnung wie üblich der $d_{50}$-Wert der Summendurchgangsverteilung in $\mu$m gewählt.

Zur vergleichenden Beurteilung der erfindungsgemäßen Pigmente mit konventionellen Pigmenten wurden die Pigmente in einer Konzentrationsreihe in einem konventionellen Nitrocellulose Lack (Erco-Bronzemischlack 2615e; erhältlich bei Fa. Rohm und Haas Deutschland, Werk Strullendorf, Reinhard-Reichnow-Str. 4, D-96129 Strullendorf) auf schwarz/weißem Kontrastpapier aufgerakelt (Rakeltiefe: 36 $\mu$m) und 24h lang bei Raumtemperatur getrocknet.

Diese Applikationen wurden optisch einerseits durch eine Glanzmessung bei jeweils 20° und 60° in Anlehnung an die DIN 67 530 (Gerät: micro-TRI-gloss von Byk-Gardner, D-82538 Geretsried, Deutschland) charakterisiert. Kalibriert wurde hier mittels Dunkelkalibrierung sowie einer schwarzen Spiegelglasplatte mit Werten von 92 für 20° und 95 für 60°. Zum anderen wurde eine farbmetrische Bestimmung der Helligkeitswerte L* bei einem konstanten Einstrahlungswinkel von 45° bei unterschiedlichen Beobachtungswinkeln (bezogen auf den Glanzwinkel) charakterisiert (Gerät: Multiflash M 45, Fa. Optronics).

Aus den Helligkeitswerten bei 15°, 45° und 110° kann nach einer ursprünglich von der Firma DuPont angegebene Formel ein Flopindex bestimmt werden, welcher gut die winkelabhängige Helligkeitsänderung von konventionellen Metallic-Applikationen wiedergibt (A.B.J. Rodriguez, JOCCA, (1992(4)) S. 150 - 153):

$$Flopindex = 2,69 \times \frac{(L^{\bullet}_{15^{\circ}} - L^{\bullet}_{110^{\circ}})^{1,11}}{(L^{\bullet}_{45^{\circ}})^{0,86}}$$

Zur Beurteilung der Deckfähigkeit der Applikationen wurde das Verhältnis der Helligkeiten L* bei einem Beobachtungswinkel von 110° auf schwarzem zu weißem Untergrund herangezogen. Betrug dieses Verhältnis > 0,98, so wird die Applikation als deckend bezeichnet. Die Messung unter einem derart flachen Beobachtungswinkel ist besonders sensitiv, da bei mangelnder Deckung der Untergrund praktisch "zwischen" den überwiegend planparallel orientierten Metallpigmenten messtechnisch erfaßt wird. Die Übereinstimmung zum visuellen Eindruck des Beobachters ist sehr gut. Üblich im Schrifttum ist hingegen die Beurteilung der Helligkeitsunterschiede **bei** diffuser Messung (z.B. in der EP 0 451 785), was jedoch zu deutlich geringerer Empfindlichkeit führt und des weiteren nicht mit dem visuellen Eindruck übereinstimmt.

Die Deckfähigkeit der Applikationen hängt entscheidend von der Konzentration der Aluminiumpigmente ab. Als ein Maß für diese Konzentration wurde eine Flächenkonzentration an Aluminiumpigmenten $c_{Al}$ in mg/cm$^2$ berechnet. Diese Größe wird anhand der Naßlackschichtdicke, die durch die Rakel vorgegeben wird, nach folgender Formel berechnet:

$$c_{Al} = 0,1 * \frac{m_{Al}}{m_{Naßlack,Al}} * \rho_{Naßlack,Al} * RH \qquad [mg/cm^2]$$

wobei:

$m_{Al}$: Einwaage Aluminiumpigmente
$m_{Naßlack;Al}$: Einwaage des Naßlackes und des Aluminiumpigmentes einschl. Lösemittel des Aluminiumpigmentes aus der Paste (i.d.R. 10,0 g)
$\rho_{Naßlack,Al}$: die Dichte des mit Aluminiumpigment vermischten Naßlacks
**RH:** die Rakelhöhe in $\mu$m ist.

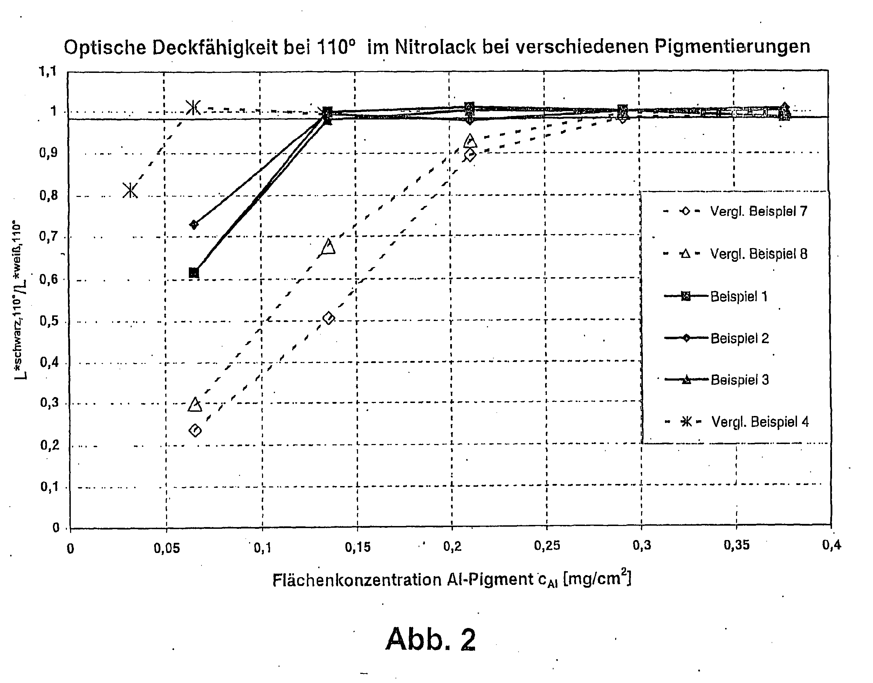

In Abb. 2 ist das optische Deckfähigkeitskriterium gegen die berechnete Flächenkonzentration an Aluminiumpigment für ausgewählte Beispiele dargestellt.

Aus Abbildung 2 ist deutlich zu erkennen, daß die erfindungsgemäßen Aluminiumpigmente eine wesentlich höhere Deckfähigkeit im Vergleich zu den konventionellen Aluminiumpigmenten aufweisen. Anhand dieser Daten wurde durch Interpolation jene Flächenkonzentration ermittelt, bei der das Verhältnis $L*_{110°,schwarz}/L*_{110°,weiß}$ 0,98 betrug.

Die erfindungsgemäßen Beispiele 1 bis 3 weisen eine hervorragende Deckfähigkeit bei einer Flächenkonzentration von weniger als 0,15 mg/cm$^2$ auf. Lediglich die über das aufwendigere PVD-Verfahren hergestellten PVD-Pigmente (Vergleichsbeispiel 4) zeigen eine bessere Deckfähigkeit.

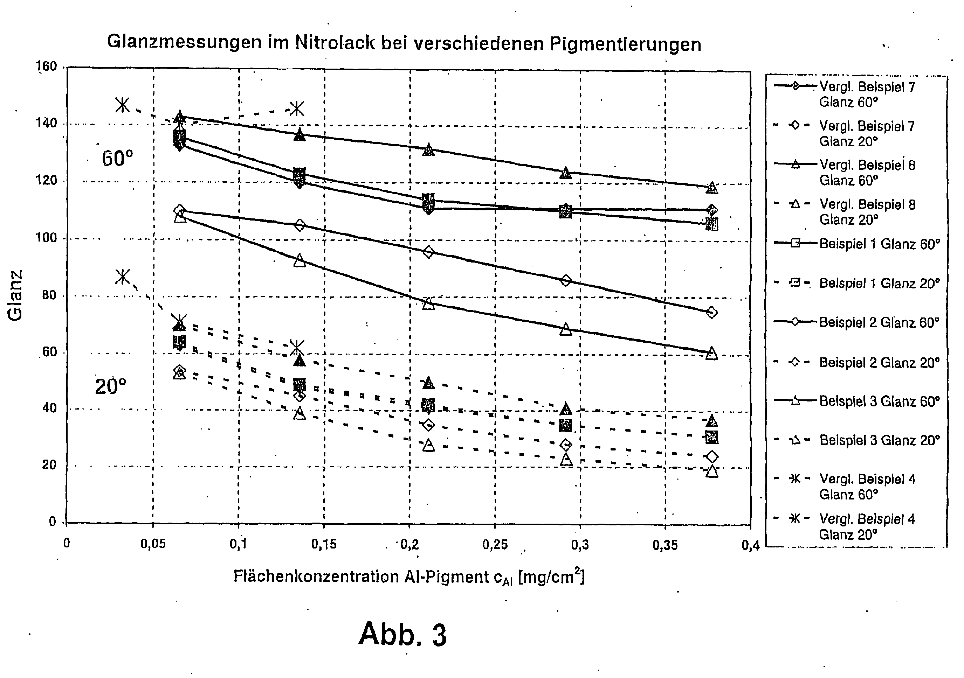

In Abb. 3 sind die gemessenen Glanzwerte bei je 60° und 20° Einfalls-/Ausfallswinkelgeometrie der Applikationen gegen die Flächenkonzentration Aluminiumpigment dargestellt. Der Glanz nimmt mit steigender Flächenkonzentration nahezu linear ab. Dies ist auf die immer schlechtere Orientierung der Metallpigmente mit steigender Pigmentierungshöhe zurückzuführen. Eine schlechtere Orientierung wird hier durch immer mehr Störungen der Stapelung der Pigmente innerhalb der Lackschicht bewirkt. Dieser Effekt ist hier besonders ausgeprägt, da die Applikation aufgrund des geringen Festkörpergehaltes des Lackes (nicht flüchtiger Anteil: ca. 6%) ein großes Al/Bindemittelverhältnis besitzt.

Aus Abb. 3 ist mithin ersichtlich, daß - abgesehen von dem PVD-Pigment Metalure® (Vergl.-Beispiel 4) - die erfindungsgemäßen Aluminiumpigmente (Beispiele 1 bis 3) bei sämtlichen Flächenkonzentrationen deutlich bessere Glanzwerte aufweisen als herkömmliche Aluminiumpigmente (Vergl.-Beispiele 7 und 8).

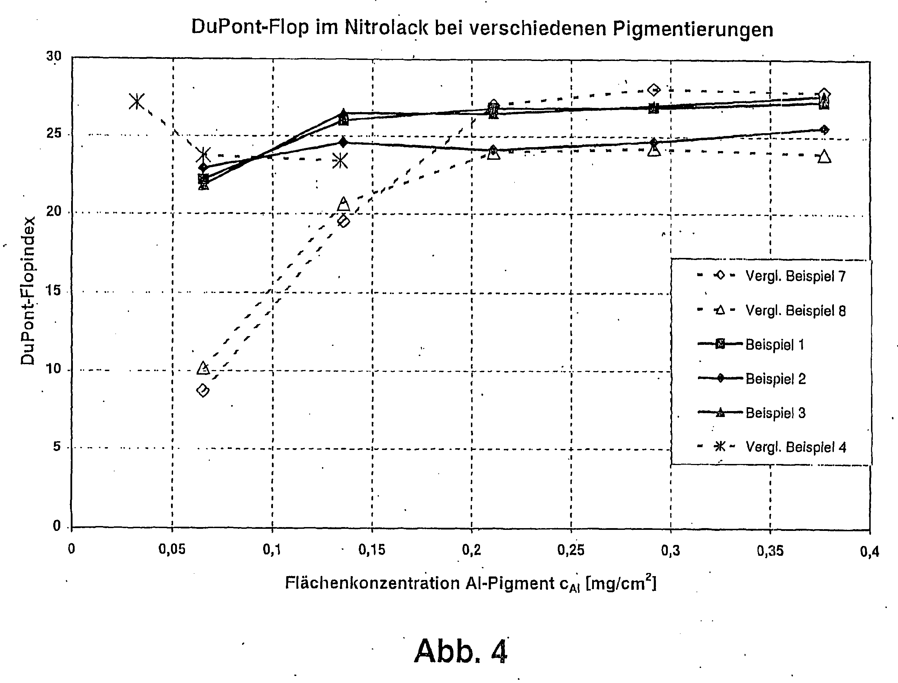

In Abb. 4 sind die Flopwerte nach DuPont gegen die Flächenkonzentration Aluminiumpigment dargestellt. Bei hohen Pigmentierungen sind die Flopwerte der erfindungsgemäßen und der konventionellen Pigmente vergleichbar. Unterhalb von ca. 0,15 mg/cm$^2$ jedoch, wenn die Deckfähigkeit der konventionellen Pigmente bereits sehr schlecht ist, machen sich deutliche Unterschiede bemerkbar. Die erfindungsgemäßen Aluminiumpigmente können aufgrund ihrer weitaus höheren Deckkraft besonders vorteilhaft in niedrigen Pigmentierungshöhen, bei denen konventionelle Pigmente nicht mehr deckfähig sind, eingesetzt werden. Hier werden Applikationen mit sehr hohem Glanz, einem guten Flop und einem stark metallischen Aussehen, d.h. einem sehr guten "Chromeffekt", zugänglich.

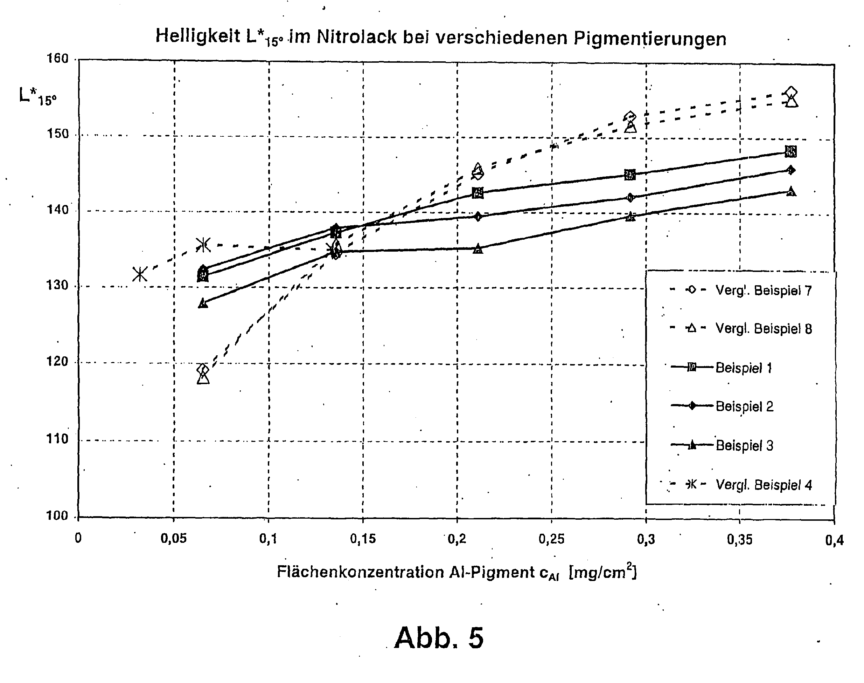

In Abb. 5 sind als ein übliches farbmetrisches Maß für die Helligkeit die L*-Werte bei 15° gegen die Flächenkonzentration Aluminiumpigment aufgetragen. Bei hohen Pigmentierungshöhen überwiegen die Werte der konventionellen Pigmente zunächst, um jedoch mit steigendem Deckungvelust deutlich abzufallen.

Dieses Ergebnis der Farbmetrik wiederspricht jedoch dem visuellen Eindruck: der Beobachter ordnet den Applikationen der erfindungsgemäßen Pigmente eine deutlich höhere Helligkeit zu als jenen der konventionellen Pigmente.

Es wird vermutet, daß diese Diskrepanz wie folgt erklärt werden kann:

Die erfindungsgemäßen Pigmente ergeben, ähnlich wie PVD-Pigmente, aufgrund ihrer geringen Dicke eine äußerst gute Orientierung und damit eine sehr hohe gerichtete Reflektion, d.h. einen hohen Glanz, daß selbst bei einem Beobachtungswinkel von 15° nahe dem Glanzwinkel der Anteil des gestreuten Lichtes bereits gering ist. Daher wird bei der farbmetrischen Beurteilung eine geringere Helligkeit suggeriert. Dies wiederspricht jedoch dem visuellen Eindruck: Applikationen konventioneller Pigmente (Vergl.-Beispiele 7 und 8) zeigen vielmehr eine deutlich "weißere", "milchigere" Helligkeit. Die erfindungsgemäßen Pigmente hingegen wirken - wie auch PVD-Pigmente (Vergl.-Beispiel 4) - deutlich metallischer in ihrem Charakter. Demnach ist auch der Flop der erfindungsgemäßen Pigmente eher höher einzustufen, als es die durch den DuPont-Flopindex ermittelten Werte suggerieren. Dies entspricht auch dem visuellen Eindruck der Applikationen.

Eine derartige Täuschung farbmetrischer Daten mag möglicherweise auch einem gemäß der Lehre der EP 0 451 785 behaupteten Zusammenhang zwischen einem Verlust der Helligkeit mit steigendem Wasserspreitungswerten und damit mit steigender Deckfähigkeit zugrunde liegen. In Fig.1 dieser Patentschrift wird ein Maximum der Helligkeit L bei einem Spreitwert von 3,2 m$^2$/g behauptet und ein deutlicher Abfall insbesondere bei Spreitwerten höher als 5 m$^2$/g suggeriert. Die Helligkeit bezieht sich jedoch nicht auf einen Meßwinkel, sondern scheint vielmehr diffus gemessen worden zu sein. Dabei wurde übersehen, daß dünnere Pigmente aufgrund der höheren gerichteten Reflektion einfallenden Lichtes notwendigerweise einen geringeren diffus gestreuten Lichtanteil besitzen müssen.

In Tab. 2 sind die aus Abb. 2 ermittelten kritischen Deckfähigkeitsflächenkonzentrationen sowie die interpolierten farbmetrischen Daten bei dieser Konzentration dargestellt.

**Tab. 2: Optische Daten bei der Deckfähigkeitsflächenkonzentration Al-Pigment $C_{Al,98\%}$**

| Probe | Deckfähigkeitsflächenkonzentration Al-Pigment $C_{Al,98\%}$ [mg/cm$^2$] | Glanz 20° bei $C_{Al,98\%}$ | Glanz 60° bei $C_{Al,98\%}$ | DuPont-Flopindex bei $C_{Al,98\%}$ | Helligkeit L*15° bei $C_{Al,98\%}$ | Genereller visueller Eindruck |
|---|---|---|---|---|---|---|
| Erf.-gem. Beispiel 1 | 0,134 | 49 | 124 | 25,8 | 137 | Sehr metallisch, "Chromeffekt" |

(fortgesetzt)

| Probe | Deckfähigkeitsflächenkonzentration Al-Pigment $C_{Al,98\%}$ [mg/cm$^2$] | Glanz 20° bei $C_{Al,98\%}$ | Glanz 60° bei $C_{Al,98\%}$ | DuPont-Flopindex bei $C_{Al,98\%}$ | Helligkeit L*15° bei $C_{Al,98\%}$ | Genereller visueller Eindruck |
|---|---|---|---|---|---|---|
| Erf.-gem. Beispiel 2 | ca. 0,12 | 52 | 123 | 24,2 | 136 | Sehr metallisch, "Chromeffekt" |
| Erf.-gem. Beispiel 3 | 0,135 | 59 | 137 | 26,4 | 134 | Sehr metallisch, "Chromeffekt" |
| Vergl.-Beispiel 7 | 0,29 | 36 | 87 | 28,2 | 153 | Metallisch |
| Vergl.-Beispiel 8 | 0,27 | 25 | 72 | 27,1 | 150 | Metallisch, relativ "weiß" |
| Vergl.-Beispiel 4 | ca. 0,05 | 79 | 144 | 25,4 | 134 | Sehr stark metallisch, "Chromeffekt" |

Ein höherer Glanz der erfindungsgemäßen Pigmente wurde auch in Naßlackapplikationen gefunden. In Tab. 3 sind die farbmetrischen Daten von Naßlackierungen ausgewählter Beispiele dargestellt. Durchweg haben die gemäß dem erfindungsgemäßen Beispiel 1 hergestellten Pigmente einen höheren Glanz als konventionelle Pigmente aus der konventionellen Naßmahlung. Jedoch ist der Glanz nicht so hoch wie bei PVD-Pigmenten (siehe Vergl.-Beispiel 4).

Die Applikationen in Tab.3 wurden durch Spritzung "auf Deckung" erstellt, d.h. deckende Pigmentierungshöhen wurden in Konzentrationsreihen ermittelt. Angegeben sind die jeweils verwendeten Pigmentierungshöhen (bezogen auf die Lackformulierung) und die gemessenen Schichtdicken der Applikationen. Die Schichtdicken wurden mit einem Qua Nix 1500 (Fa. Lau GmbH, D-58675 Hemer, Deutschland) gemessen. Deutlich wird auch hier die höhere Deckkraft und damit verbunden auch geringere Schichtdicken der erfindungsgemäßen Pigmente im Vergleich zu konventionellen Pigmenten. Auch hier jedoch hat ein PVD-Pigment eine noch bessere Deckfähigkeit und stärker metallische Eigenschaften.

**Tab. 3:** Naßlackapplikationen ohne Klarlack bei verschiedenen Schichtdicken und Pigmentierungen auf Deckung gespritzt. Lacksystem: 2K-Chromeffektlack mit niedrigem Festkörpergehalt. ("Metalure" Brochure, Fa Eckart).

| Probe | Glanz | | DuPont-Flopindex | Pigmentierung (bez. auf Formylierung) | Schichtdicke |
|---|---|---|---|---|---|
| | 60° | 20° | | | |
| Erf.-gem. Beispiel 1 | 84 | 29 | 24,0 | 1,8%. | 4-6 μm |
| Metalure L 55700 (Vergl. Beispiel 4) | 128 | 57 | 24,6 | 1,5% | 2-3 μm |
| VP 53534 (Vergl. Beispiel 7) | 74,4 | 25 | 24,5 | 4% | 6-8 μm |

**Beispiele zur Passivierung der erfindungsgemäßen Aluminiumpigmente:**

Beispiel 9: (SiO$_2$- beschichtetes Aluminium)

55,1 g einer Paste mit Aluminiumpigmenten nach Beispiel 1 (entspricht 38,5 g Al) werden in 375 ml Isopropanol dispergiert und auf Siedetemperatur gebracht. 13,35 g Tetraethoxysilan werden zugegeben. Anschließend dosiert man über einen Zeitraum von 3 h eine Lösung von 5,4 g 25 %-igen NH$_3$ in 9,3 g Wasser hinzu. Nach weiteren 3 h wird auf Raumtemperatur abgekühlt und die Suspension über einen

Büchnertrichter abgenutscht. Anschließend wird das Produkt über Nacht in einem Vakuumtrockenschrank bei 100°C getrocknet.

Beispiel 10: (Chromatiertes Aluminium)

18g einer Chromsäurelösung werden hergestellt, indem man 4,5g $CrO_3$ in 13,5 g Wasser (vollentsalzt) auflöst.
In einem 1 L-Reaktor werden 220g Wasser (vollentsalzt)auf 90°C aufgeheizt. Unter heftigem Rühren (Rühraggregat: Stollenscheibe) gibt man zunächst 21g Butylglykol und anschließend 125g des in Beispiel 1 beschriebenen Aluminiumpigmentes in Form einer Testbenzinpaste mit einem Festkörperanteil von 70%, hinzu. Wenige Minuten später erfolgt die Zugabe der Chromsäurelösung bei einer Reaktionstemperatur von 80°C. Man läßt unter starkem Rühren das Gemisch weitere 50 min lang reagieren. Dann läßt man 30 min lang abkühlen und dekantiert in einem Becherglas das Reaktionsgemisch mehrmals mit je 250ml einer 5%igen VE-$H_2O$/Butylglykol-Lsg. bis keine Gelbfärbung der überstehenden Lösung mehr auftritt. Anschließend wird das Produkt auf einer Nutsche abfiltriert und mit viel Wasser (ca. 3L) gewaschen.

Gasungstest:

8,6 g Al werden in Form einer Paste 315 g farblosen Wasser-Mischlack (ZW42-1100, Fa. BASF Würzburg) eingearbeitet und mit Dimethanolethanolamin auf pH 8,2 gebracht. Von diesem Lack werden 300 g in eine Gaswaschflasche eingefüllt und diese mit einem Doppelkammergasblasenzähler verschlossen. Die Gasmenge kann anhand der verdrängten Wassermenge in der unteren Kammer des Gasblasenzählers abgelesen werden. Die Gaswaschflasche wird in einem Wasserbad bei 40°C temperiert und der Test über 30 Tage durchgeführt. Er gilt als bestanden, wenn nach 7 d nicht mehr als 4 und nach 30 Tagen nicht mehr als 20 ml Wasserstoff entwickelt wurden.

**Tab. 4:** Ergebnisse Gasungstest von beschichteten dünnen Aluminiumpigmenten

| Probe | Gasung nach 7d | Gasung nach 30 d |
|---|---|---|
| Beispiel 9 | 2 ml | 8 ml |
| Beispiel 10 | 1 ml | 5 ml |
| Vergl. Beispiel 11 (unbeschichtetes Pigment nach Bsp. 1) | < 3 h !! | -- |
| d: Tage | | |

Aus Tabelle 4 ist zu entnehmen, daß die erfindungsgemäßen Aluminiumpigmente hervorrragend gegenüber Korrosion stabilisiert werden können.
Das erfindungsgemäße Verfahren ermöglicht die Herstellung von Aluminiumpigmenten, die in ihren physikalischen Eigenschaften den PVD-Pigmenten sehr nahe kommen, jedoch auf wesentlich einfachere Art und Weise hergestellt werden können. Gegenüber den konventionellen Aluminiumpigmenten weisen die erfindungsgemäßen Aluminiumpigmente, insbesondere im Hinblick auf die Deckfähigkeit und den Glanz deutlich verbesserte Eigenschaften auf. Schließlich weisen die erfindungsgemäßen Aluminiumpigmente nicht die bei PVD-Pigmenten nachteilige Agglomerationsneigung auf. Die erfindungsgemäßen Aluminiumpigmente können daher in Präparationen, die beispielsweise Lacksystemen, Druckfarben oder Kosmetika, zugesetzt werden, deutlich höher konzentriert werden. Dies erleichtert die Handhabung wesentlich.
Die erfindungsgemäßen Aluminiumpigmente vereinigen daher die vorteilhaften Eigenschaften von herkömmlichen Aluminiumpigmenten, insbesondere die einfache Herstellbarkeit und Handhabbarkeit, und von PVD-Pigmenten, insbesondere die hohe Deckfähigkeit, die hohen Glanzeigenschaften und ein stark metallisches Aussehen.

**Patentansprüche**

1. Aluminiumpigmente, welche wenigstens teilweise mit Schmiermittel belegt sind,
**dadurch gekennzeichnet,**
**daß** die Aluminiumpigmente

a) einen Wasser-Spreitwert zwischen 40.000 bis 130.000 cm$^2$/g,
b) eine aus dem Wasser-Spreitwert sowie eine über Dickenauszählung mit Rasterelektronenmikroskopie aus

dem $h_{50}$-Wert der Summendurchgangsverteilung errechnete mittlere Dicke h von unter 100 bis 30 nm,
c) eine über Dickenauszählung mit Rasterelektronenmikroskopie ermittelte relative Breite der Dickenverteilung $\Delta$h, welche anhand der entsprechenden Summendurchgangskurve der relativen Häufigkeiten nach der Formel

$$\Delta h = 100 \times \frac{h_{90} - h_{10}}{h_{50}}$$ berechnet wird, von 70 % bis 140 %,

d) einen Formfaktor $d_{50}$/h von über 200, wobei der $d_{50}$-Wert 50% der Durchgangssumenverteilungskurve der Längsausdehnung d, gemessen und ausgewertet in Form einer Volumenverteilung von Equivalentkugeln entspricht.
e) einen Rauwert, der sich aus der spezifischen Oberfläche, welche nach der BET-Methode gemessen wird, und dem Spreitwert nach Maßgabe der folgenden Formel

BET-Wert/ (2 x Spreitwert) berechnet wird, von 0,30 bis 0,9 aufweisen.

**2.** Aluminiumpigmente nach Anspruch 1,
**dadurch gekennzeichnet,**
**daß** die Aluminiumpigmente eine über Dickenauszählung mit Rasterelektronenmikroskopie ermittelte relative Breite der Dickenverteilung $\Delta$h, welche anhand der entsprechenden Summendurchgangskurve der relativen Häufigkeiten

nach der Formel $\Delta h = 100 \times \dfrac{h_{90} - h_{10}}{h_{50}}$ berechnet wird,

**3.** Aluminiumpigmente nach einem der vorherigen Ansprüche,
**dadurch gekennzeichnet,**
**daß** die Aluminiumpigmente einen Formfaktor $d_{50}$/h über 220 aufweisen.

**4.** Aluminiumpigmente nach einem der vorherigen Ansprüche,
**dadurch gekennzeichnet,**
**daß** die Alumiumpigmente einen Rauwert, der sich aus der spezifischen Oberfläche, welche nach der BET-Methode gemessen wird, und dem Spreitwert nach Maßgabe der folgenden Formel BET-Wert/2 x Spreitwert berechnet wird, von 0,35 bis 0,9 aufweisen.

**5.** Aluminiumpigmente nach einem der vorherigen Ansprüche,
**dadurch gekennzeichnet,**
**daß** die Aluminiumpigmente wenigstens teilweise mit Fettsäuren als Schmiermittel belegt sind.

**6.** Aluminiumpigmente nach einem der vorherigen Ansprüche,
**dadurch gekennzeichnet,**
**daß** die Aluminiumpigmente wenigstens teilweise mit Stearinsäure als Schmiermittel belegt sind.

**7.** Aluminiumpigmente nach einem der Ansprüche 1 bis 5,
**dadurch gekennzeichnet,**
**daß** die Aluminiumpigmente wenigstens teilweise mit Ölsäure als Schmiermittel belegt sind.

**8.** Aluminiumpigmente nach einem der Ansprüche 1 bis 5,
**dadurch gekennzeichnet,**
**daß** die Aluminiumpigmente wenigstens teilweise mit einem Gemisch aus Stearinsäure und Ölsäure als Schmiermittel belegt sind.

**9.** Aluminiumpigmente nach einem der Ansprüche 1 bis 5,
**dadurch gekennzeichnet,**
**daß** die Aluminiumpigmente wenigstens teilweise mit Phosphonsäuren, Phosphorsäureestern oder einem Gemisch davon als Schmiermittel belegt sind.

**10.** Aluminiumpigmente nach einem der vorherigen Ansprüche,
**dadurch gekennzeichnet**,
das die Aluminiumpigmente mit einer passivierenden Inhibitor- oder Korrosionsschutzschicht belegt sind.

**11.** Aluminiumpigmente nach Anspruch 10,
**dadurch gekennzeichnet,**
**daß** die passivierende Inhibitorschicht korrosionsinhibierende organische Phosphonsäuren und/oder Phosphorsäureester, organisch funktionalisierte Silane, aliphatische oder cyclische Amine, aliphatische oder aromatische Nitroverbindungen, Sauerstoff-, Schwefel- und/oder Stickstoff-enthaltende Heterocyclen, Schwefel-und/oder Stickstoffverbindungen höherer Ketone, Aldehyde und Alkohole, Thiole, β-Ketoester, β-Diketone oder Gemischen derselben umfaßt.

**12.** Aluminiumpigmente nach Anspruch 10,
**dadurch gekennzeichnet**,
das die passivierende Korrosionsschutzschicht Siliciumoxid, Zirkoniumoxid, Aluminiumoxid, Chromoxid, polymerisierte Kunststoffharze, Vanadiumoxide, Molybdänoxide und/oder-peroxide, Phosphate, Phosphite, Borate oder Mischungen derselben umfaßt.

**13.** Aluminiumpigment nach Anspruch 10,
**dadurch gekennzeichnet**,
das die passivierende Korrosionsschutzschicht Siliciumdioxid umfaßt, wobei die Siliciumdioxid-Oberfläche vorzugsweise mit Silanen belegt ist.

**14.** Aluminiumpigmente nach einem der Ansprüche 1 bis 9,
**dadurch gekennzeichnet,**
**daß** die Aluminiumpigmente naßchemisch durch Wasser oxidiert sind und die Aluminiumpigmente ein farbiges Aussehen aufweisen.

**15.** Aluminiumpigmente nach einem der vorherigen Ansprüche,
**dadurch gekennzeichnet,**
**daß** die Aluminiumpigmente als Pulver, vorzugsweise nicht-staubendes Pulver, oder in kompaktierter Form, vorzugsweise als Paste, Granulat oder Pellets vorliegen.

**16.** Verfahren zur Herstellung von Aluminiumpigmenten nach einem der Ansprüche 1 bis 15 das den folgenden Schritt umfaßt:

a) Vermahlen von Aluminiumpartikeln mit einem mittleren Durchmesser von unter 10 $\mu$m unter Verwendung eines Mahlwerks, wobei das Mahlwerk eine Kugelmühle oder Topfmühle ist, in Gegenwart von Lösemittel und Shmierstoffen und Mahlkugeln, die ein Einzelgewicht von 2 bis 13 mg aufweisen, über einen Zeitraum von 15 bis 72 Stunden zu Aluminiumpigmenten, wobei die Umdrehungsgeschwindigkeit des Mahlwertes 50 bis 62% der kritischen Drehzahl $n_{Krit}$ beträgt.

**17.** Verfahren nach Anspruch 16,
**dadurch gekennzeichnet,**
**daß** die Mahlkörper ein Einzelgewicht von 5,0 bis 12 mg aufweisen.

**18.** Verfahren nach Anspruch 16 oder 17,
**dadurch gekennzeichnet,**
**daß** die Aluminiumpigmente in einem weiteren Schritt b) einer Größenklassifikation unterzogen werden.

**19.** Verfahren nach einem der Ansprüche 16 bis 18,
**dadurch gekennzeichnet,**
**daß** die in Schritt a) oder b) bereitgestellten Aluminiumpigmente in eine kompaktierte Form, vorzugsweise Paste, Granulat oder Pellets, überführt werden.

**20.** Verfahren nach einem der Ansprüche 16 bis 18,
**dadurch gekennzeichnet,**
**daß** die in Schritt a) oder b) bereitgestellten Aluminiumpigmente in ein Aluminiumpulver, vorzugsweise ein nichtstaubendes Aluminiumpulver, überführt werden.

**21.** Verfahren nach einem der Ansprüche 16 bis 20,
**dadurch gekennzeichnet,**

**daß** als Lösemittel organische Lösemittel, vorzugsweise Testbenzin, Solventnaphta, Isopropanol, Alkohole, Ketone oder Mischungen davon, verwendet werden.

22. Verfahren nach einem der Ansprüche 16 bis 21,
**dadurch gekennzeichnet,**
**daß** als Lösemittel Wasser und als Schmierstoffe organische Phosphonsäuren, und/oder -ester und/oder Phosphorsäuren und/oder - ester verwendet werden.

23. Verwendung von Aluminiumpigmenten nach einem der Ansprüche 1 bis 15 in Coatings, Lacken, Druckfarben, Pulverlacken, Kunststoffen, Sicherheitsdrucken, Keramiken und kosmetischen Formulierungen, vorzugsweise Nagellack.

24. Verwendung von beschichteten Aluminiumpigmenten nach einem der Ansprüche 10 bis 12, in Wasserlacken und Beschichtungsmitteln für Außenanwendungen.

25. Nagellack,
**dadurch gekennzeichnet,**
**daß** der Nagellack Aluminiumpigmente nach einem der Ansprüche 1 bis 15 enthält.

26. Wasserlack,
**dadurch gekennzeichnet,**
**daß** der Wasserlack Aluminiumpigmente nach einem der Ansprüche 9 bis 14 enthält.

**Claims**

1. Aluminium pigments which are at least partially coated with lubricant,
**characterised in that**
the aluminium pigments

a) have a water-spreading value of between 40,000 and 130,000 $cm^2/g$,
b) a mean thickness h of less than 100 to 30 nm, calculated from the water-spreading value and by counting the thickness from the $h_{50}$ value of the cumulative intensity profile using scanning electron microscopy,
c) a relative range of thickness distribution $\Delta h$ of 70 % to 140 %, determined by counting the thickness using scanning electron microscopy, which is calculated from the corresponding cumulative intensity curve of the relative frequencies using the formula

$$\Delta h = 100 \times \frac{h_{90} - h_{10}}{h_{50}}$$

d) a form factor $d_{50}/h$ of more than 200, where the $d_{50}$ value corresponds to 50 % of the cumulative intensity distribution curve of the longitudinal extension d, measured and evaluated in the form of a distribution by volume of equivalent spheres,
e) a peak-to-valley value of from 0.30 to 0.9, calculated from the specific surface measured by the BET method and the spreading value based on the following formula BET value (2 x spreading value).

2. Aluminium pigments as claimed in claim 1,
**characterised in that**
the aluminium pigments have a relative range of thickness distribution $\Delta h$ of 75 % to 120 % determined by counting the thickness by scanning electron microscopy, which is calculated from the corresponding cumulative intensity curve of the relative frequencies based on the formula

$$\Delta h = 100 \times \frac{h_{90} - h_{10}}{h_{50}}$$

**3.** Aluminium pigments as claimed in one of the preceding claims,
**characterised in that**
the aluminium pigments have a form factor $d_{50}/h$ of more than 220.

**4.** Aluminium pigments as claimed in one of the preceding claims,
**characterised in that**
the aluminium pigments have a peak-to-valley factor of 0.35 to 0.9, calculated from the specific surface measured by the BET method and the spreading value on the basis of the formula BET value/2 x spreading value.

**5.** Aluminium pigments as claimed in one of the preceding claims,
**characterised in that**
the aluminium pigments are at least partially coated with fatty acids as lubricants.

**6.** Aluminium pigments as claimed in one of the preceding claims,
**characterised in that**
the aluminium pigments are at least partially coated with stearic acid as lubricant.

**7.** Aluminium pigments as claimed in one of claims 1 to 5,
**characterised in that**
the aluminium pigments are at least partially coated with oleic acid as lubricant.

**8.** Aluminium pigments as claimed in one of claims 1 to 5,
**characterised in that**
the aluminium pigments are at least partially coated with a mixture of stearic acid and oleic acid as lubricant.

**9.** Aluminium pigments as claimed in one of claims 1 to 5,
**characterised in that**
the aluminium pigments are at least partially coated with phosphonic acids, phosphoric acid esters or a mixture thereof as lubricant.

**10.** Aluminium pigments as claimed in one of the preceding claims,
**characterised in that**
the aluminium pigments are coated with a passivating inhibitor or anti-corrosion coating.

**11.** Aluminium pigments as claimed in claim 10,
**characterised in that**
the passivating inhibitor coating contains corrosion-inhibiting, organic phosphonic acids and/or phosphoric acid esters, organically functionalised silanes, aliphatic or cyclic amines, aliphatic or aromatic nitro-compounds, heterocycles containing oxygen, sulphur and/or nitrogen, higher ketones containing sulphur and/or nitrogen compounds, aldehydes and alcohols, thiols, β-keto-esters, β-diketones or mixtures thereof.

**12.** Aluminium pigments as claimed in claim 10,
**characterised in that**
the passivating anti-corrosion coating contains silicium dioxide, zirconium dioxide, aluminium oxide, chromium oxide, polymerised synthetic resins, vanadium oxides, molybdenum oxides and/or peroxides, phosphates, phosphites, borates or mixtures thereof.

**13.** Aluminium pigment as claimed in claim 10,
**characterised in that**
the passivating anti-corrosion coating contains silicium dioxide, and the silicium dioxide surface is preferably coated with silanes.

**14.** Aluminium pigments as claimed in one of claims 1 to 9,
**characterised in that**
the aluminium pigments are oxidised by means of water in a wet chemical process and the aluminium pigments have a coloured appearance.

**15.** Aluminium pigments as claimed in one of the preceding claims,
**characterised in that**
the aluminium pigments are produced as a powder, preferably a powder that does not generate dust, or in compacted form, preferably as a paste, granulate or pellets.

**16.** Method of producing aluminium pigments as claimed in one of claims 1 to 15, comprising the following steps:

a) grinding aluminium particles with a mean diameter of less then 10 $\mu$m using a mill, which mill is a ball mill or pot mill, in the presence of solvents and lubricants and grinding balls with an individual weight of 2 to 13 mg over a period of from 15 to 72 hours to produce aluminium pigments, and the mill is operated at a rotation speed of 50 to 62 % of the critical speed $n_{crit}$.

**17.** Method as claimed in claim 16,
**characterised in that**
the grinding balls have an individual weight of 5.0 to 2 mg.

**18.** Method as claimed in claim 16 or 17,
**characterised in that**
the aluminium pigments are subjected to a size classification in another step b).

**19.** Method as claimed in one of claims 16 to 18,
**characterised in that**
the aluminium pigments produced in step a) or b) are processed to a compacted form, preferably paste, granulate or pellets.

**20.** Method as claimed in one of claims 16 to 18,
**characterised in that**
the aluminium pigments produced in step a) or b) are processed to an aluminium powder, preferably an aluminium powder that does not generate dust.

**21.** Method as claimed in one of claims 16 to 20,
**characterised in that**
the solvents used are organic solvents, preferably test benzene, solvent naphtha, isopropanol, alcohols, ketones or mixtures thereof.

**22.** Method as claimed in one of claims 16 to 21,
**characterised in that**
water is used as a solvent and organic phosphonic acids and/or esters and/or phosphoric acids and/or esters are used as lubricants.

**23.** Use of aluminium pigments as claimed in one of claims 1 to 15 in coatings, varnishes, paints, powdered varnishes, plastics, safety inks, ceramics and cosmetic formulations, preferably nail varnish.

**24.** Use of coated aluminium pigments as claimed in one of claims 10 to 12 in water-based varnishes and coating agents for outdoor applications.

**25.** Nail varnish,
**characterised in that**
the nail varnish contains aluminium pigments as claimed in one of claims 1 to 15.

**26.** Water-based varnish,
**characterised in that**
the water-based varnish contains aluminium pigments as claimed in one of claims 9 to 14.

**EP 1 613 702 B1**

**Revendications**

1. Pigments d'aluminium qui sont revêtus au moins en partie d'agent lubrifiant, **caractérisés en ce que** les pigments d'aluminium présentent

   a) une valeur de dispersion dans l'eau entre 40 000 et 130 000 $cm^2/g$,
   b) une épaisseur moyenne h calculée à partir de la valeur de dispersion dans l'eau et calculée par comptage de l'épaisseur par microscopie à balayage électronique à partir de la valeur $h_{50}$ de la répartition de passage totale, inférieure à 100 à 30 nm,
   c) une largeur relative déterminée par comptage de l'épaisseur par microscopie à balayage électronique de la répartition des épaisseurs Δh qui est calculée sur la base des courbes de passage totales correspondantes des fréquences relatives selon la formule

$$\Delta h = 100 \ \times \ \frac{h_{90} - h_{10}}{h_{50}},$$

   de 70 % à 140 %,
   d) un facteur de forme $d_{50}/h$ supérieur à 200, la valeur $d_{50}$ correspondant à 50 % des courbes de répartition de passage totale de l'extensibilité en suspension d, mesuré et évalué sous forme d'une répartition volumique de billes équivalentes,
   e) une valeur brute qui est mesurée à partir de la surface spécifique qui est mesurée selon le procédé BET et de la valeur de dispersion selon la formule suivante : valeur BET (2 x valeur de dispersion), de 0,30 à 0,9.

2. Pigments d'aluminium selon la revendication 1,
   **caractérisés en ce**
   **que** les pigments d'aluminium présentent une largeur relative déterminée par comptage de l'épaisseur par microscopie à balayage électronique de la répartition des épaisseurs Δh qui est calculée sur la base des courbes totales correspondantes des fréquences relatives selon la formule

$$\Delta h = 100 \ \times \ \frac{h_{90} - h_{10}}{h_{50}},$$

   de 75 % à 120 %.

3. Pigments d'aluminium selon l'une des revendications précédentes,
   **caractérisés en ce**
   **que** les pigments d'aluminium présentent un facteur de forme $d_{50}/h$ supérieur à 220.

4. Pigments d'aluminium selon l'une des revendications précédentes,
   **caractérisés en ce**
   **que** les pigments d'aluminium présentent une valeur brute qui est calculée à partir de la surface spécifique mesurée selon le procédé BET et de la valeur de dispersion selon la formule suivante valeur BET (2 x valeur de dispersion), de 0,35 à 0,9.

5. Pigments d'aluminium selon l'une des revendications précédentes,
   **caractérisés en ce**
   **que** les pigments d'aluminium sont revêtus au moins en partie avec des acides gras en tant qu'agents lubrifiants.

6. Pigments d'aluminium selon l'une des revendications précédentes,
   **caractérisés en ce**
   **que** les pigments d'aluminium sont revêtus au moins partiellement d'acide stéarique en tant qu'agent lubrifiant.

7. Pigments d'aluminium selon l'une des revendications 1 à 5,
   **caractérisés en ce**

**que** les pigments d'aluminium sont revêtus au moins partiellement d'acide oléique en tant qu'agent lubrifiant.

8. Pigments d'aluminium selon l'une des revendications 1 à 5,
**caractérisés en ce**
**que** les pigments d'aluminium sont revêtus au moins partiellement d'un mélange d'acide stéarique et d'acide oléique en tant qu'agent lubrifiant.

9. Pigments d'aluminium selon l'une des revendications 1 à 5,
**caractérisés en ce**
**que** les pigments d'aluminium sont revêtus au moins partiellement d'acides phosphoriques, d'esters d'acide phosphorique ou d'un mélange de ceux-ci en tant qu'agent lubrifiant.

10. Pigments d'aluminium selon l'une des revendications précédentes,
**caractérisés en ce**
**que** les pigments d'aluminium sont revêtus d'une couche inhibitrice passivante ou d'une couche de protection contre la corrosion.

11. Pigments d'aluminium selon la revendication 10,
**caractérisés en ce**
**que** la couche inhibitrice passivante comprend des acides phosphoniques organiques et/ou des esters phosphoriques inhibant la corrosion, des silanes à fonctionnalité organique, des amines aliphatiques ou cycliques ou des composés nitro aliphatiques ou aromatiques, des hétérocycles oxygénés, soufrés et/ou azotés, des composés de soufre et/ou d'azote de cétones supérieures, des aldéhydes et alcools, des thiols, des β-céto-esters, des β-dicétones ou des mélanges de ceux-ci.

12. Pigments d'aluminium selon la revendication 10,
**caractérisés en ce**
**que** la couche passivante de protection contre la corrosion comprend de l'oxyde de silicium, de l'oxyde de zirconium, de l'oxyde d'aluminium, de l'oxyde de chrome, des résines plastiques polymérisées, des oxydes de vanadium, des oxydes et/ou peroxydes de molybdène, des phosphates, phosphites, borates ou des mélanges de ceux-ci.

13. Pigments d'aluminium selon la revendication 10,
**caractérisés en ce**
**que** la couche passivante de protection contre la corrosion comprend du dioxyde de silicium, la surface de dioxyde de silicium étant revêtue de préférence de silanes.

14. Pigments d'aluminium selon l'une des revendications 1 à 9,
**caractérisés en ce**
**que** les pigments d'aluminium sont oxydés chimiquement à l'état humide par l'eau et les pigments d'aluminium présentent un aspect coloré.

15. Pigments d'aluminium selon l'une des revendications précédentes,
**caractérisés en ce**
**que** les pigments d'aluminium se présentent sous forme de poudre, de préférence de poudre ne formant pas de poussières ou sous forme compacte, de préférence en pâte, granulés ou pastilles.

16. Procédé de production de pigments d'aluminium selon l'une des revendications 1 à 15 qui comprend l'étape suivante :

a) le broyage de particules d'aluminium présentant un diamètre moyen inférieur à 10 μm en utilisant un broyeur, le broyeur étant un broyeur à billes ou un broyeur à jarre en présence de solvants et d'agents lubrifiants et des billes de broyage qui présentent un poids respectif de 2 à 13 mg, pendant une durée de 15 à 72 heures en pigments d'aluminium, la vitesse de rotation du broyeur étant de 50 à 62 % de la vitesse de rotation critique.

17. Procédé selon la revendication 16,
**caractérisé en ce**
**que** les corps de broyage présentent un poids respectif de 5,0 à 12 mg.

18. Procédé selon la revendication 16 ou 17,

**caractérisé en ce**
**que** les pigments d'aluminium sont soumis dans une autre étape b) à une classification de tailles.

19. Procédé selon l'une des revendications 16 à 18,
**caractérisé en ce**
**que** les pigments d'aluminium proposés à l'étape a) ou b) sont convertis en une forme compactée, de préférence une pâte, des granulés ou des pastilles.

20. Procédé selon l'une des revendications 16 à 18,
**caractérisé en ce**
**que** les pigments d'aluminium proposés à l'étape a) ou b) sont convertis en une poudre d'aluminium, de préférence une poudre d'aluminium ne formant pas de poussières.

21. Procédé selon l'une des revendications 16 à 20,
**caractérisé en ce**
**que** l'on utilise comme solvants, des solvants organiques, de préférence, le white spirit, le solvant de naphta, l'isopropanol, les alcools, cétones ou leurs mélanges.

22. Procédé selon l'une des revendications 16 à 21,
**caractérisé en ce**
**que** l'on utilise comme solvant, de l'eau et comme agents lubrifiants, des acides et/ou esters phosphoniques organiques et/ou des acides et/ou esters phosphoriques.

23. Utilisation de pigments d'aluminium selon l'une des revendications 1 à 15 dans les revêtements, les laques, les encres d'impression, les laques en poudre, les matières plastiques, les impressions de sécurité, les céramiques et les formulations cosmétiques, de préférence, le vernis à ongles.

24. Utilisation de pigments d'aluminium revêtus selon l'une des revendications 10 à 12, dans les laques à l'eau et les revêtements pour les utilisations extérieures.

25. Vernis à ongles,
**caractérisé en ce**
**que** le vernis à ongles contient des pigments d'aluminium selon l'une des revendications 1 à 15.

26. Laque à l'eau,
**caractérisée en ce**
**que** la laque à l'eau contient des pigments d'aluminium selon l'une des revendications 9 à 14.

Summendurchgangskurven der Dickenverteilungen ermittelt mit REM

Abb. 1a

EP 1 613 702 B1

Summendurchgangskurven der Dickenverteilungen ermittelt mit REM
Kleine Dickenskala

Abb. 1b

Dicke / nm

Summendurchgangsverteilung rel. Häufigkeiten

Beispiel 1
Beispiel 2
Vergl. Beispiel 4
Vergl. Beispiel 5
Vergl. Beispiel 6
Vergl. Beispiel 7

26

Optische Deckfähigkeit bei 110° im Nitrolack bei verschiedenen Pigmentierungen

Abb. 2

Glanzmessungen im Nitrolack bei verschiedenen Pigmentierungen

**Abb. 3**

Legend:
- —◇— Vergl. Beispiel 7 Glanz 60°
- - ◇ - Vergl. Beispiel 7 Glanz 20°
- —△— Vergl. Beispiel 8 Glanz 60°
- - △ - Vergl. Beispiel 8 Glanz 20°
- —⊟— Beispiel 1 Glanz 60°
- - ⊟ - Beispiel 1 Glanz 20°
- —◇— Beispiel 2 Glanz 60°
- - ◇ - Beispiel 2 Glanz 20°
- —△— Beispiel 3 Glanz 60°
- - △ - Beispiel 3 Glanz 20°
- - ✳ - Vergl. Beispiel 4 Glanz 60°
- - ✳ - Vergl. Beispiel 4 Glanz 20°

Axis: Glanz vs Flächenkonzentration Al-Pigment $c_{Al}$ [mg/cm$^2$]

EP 1 613 702 B1

DuPont-Flop im Nitrolack bei verschiedenen Pigmentierungen

Legend:
- ◇ - Vergl. Beispiel 7
- △ - Vergl. Beispiel 8
- Beispiel 1
- Beispiel 2
- Beispiel 3
- ✳ - Vergl. Beispiel 4

Y-axis: DuPont-Flopindex

X-axis: Flächenkonzentration Al-Pigment $c_{Al}$ [mg/cm²]

Abb. 4

EP 1 613 702 B1

Helligkeit L*$_{15°}$ im Nitrolack bei verschiedenen Pigmentierungen

Flächenkonzentration Al-Pigment c$_{Al}$ [mg/cm$^2$]

## Abb. 5

EP 1 613 702 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0451785 B2
- EP 0305158 A
- EP 1424371 A
- EP 1080810 A
- US 4318747 A
- US 3776473 A
- DE 19635085
- DE 10001437
- EP 0451785 A

**In der Beschreibung aufgeführte Nicht-Patentliteratur**

- **J. Seubert ; A. Fetz.** PVD Aluminium Pigments: Superior Brillance for Coatings and Graphic Arts. *Coatings Journal,* Juli 2001, vol. 84, A6 2, 240-245
- **A.B.J. Rodriguez.** *JOCCA,* 1992, 150-153